(19) Europäisches Patentamt
European Patent Office
Office européen des brevets

(11) **EP 2 384 125 B1**

(12) **EUROPEAN PATENT SPECIFICATION**

(45) Date of publication and mention
of the grant of the patent:
**17.07.2013 Bulletin 2013/29**

(51) Int Cl.:
*A23J 3/34* (2006.01)    *A23L 1/305* (2006.01)
*A23L 1/29* (2006.01)

(21) Application number: **09797247.5**

(86) International application number:
**PCT/US2009/069867**

(22) Date of filing: **30.12.2009**

(87) International publication number:
**WO 2010/078461 (08.07.2010 Gazette 2010/27)**

(54) **PROTEIN HYDROLYSATE COMPOSITIONS HAVING ENHANCED CCK RELEASING ABILITY**

PROTEINHYDROLYSATZUSAMMENSETZUNGEN MIT ERHÖHTER CCK-FREISETZUNGSFÄHIGKEIT

COMPOSITIONS D'HYDROLYSAT DE PROTEINE PRESENTANT UNE APTITUDE A LA LIBERATION DE CHOLECYSTOKININE AMELIOREE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **31.12.2008 US 141931 P**

(43) Date of publication of application:
**09.11.2011 Bulletin 2011/45**

(60) Divisional application:
**13159079.6 / 2 604 125**

(73) Proprietors:
• **Solae, Llc**
**St. Louis, MO 63110 (US)**
• **Novozymes A/S**
**2880 Bagsvaerd (DK)**

(72) Inventors:
• **KRUL, Elaine**
**Warson Woods**
**Missouri 63122 (US)**
• **WONG, Theodore M.**
**Ballwin**
**Missouri 63021 (US)**
• **LOMBARDI, Jason F.**
**Edwardsville**
**Illinois 62025 (US)**
• **SCHASTEEN, Charles S.**
**University City**
**Missouri 63130 (US)**

(74) Representative: **Hirsch & Associés**
**58, avenue Marceau**
**75008 Paris (FR)**

(56) References cited:
**WO-A1-2004/002241**    **WO-A1-2007/064208**

• **CORDIER-BUSSAT, M. ET AL.: "Peptones Stimulate Cholecystokinin Secretion and Gene Transcription in the Intestinal Cell Line STC-1" ENDOCRINOLOGY, [Online] vol. 138, no. 3, 1997, pages 1137-1144, XP002577475 Retrieved from the Internet: URL:http://endo.endojournals.org/cgi/conte nt/full/138/3/1137> [retrieved on 2010-04-12]**
• **TAKASHI NISHI, HIROSHI HARA, KOZO ASANO AND FUSAO TOMITA: "The Soybean Beta-Conglycin Beta-51-63 Fragment Suppresses Appetite by Stimulating Cholecystokinin Release in Rats" THE JOURNAL OF NUTRITION, [Online] 2003, pages 2537-2542, XP002577476 Retrieved from the Internet: URL:http://jn.nutrition.org/cgi/content/fu ll/133/8/2537> [retrieved on 2010-04-12]**
• **JELENA PUPOVAC AND G. HARVEY ANDERSON: "Dietary Peptides Induce Satiety via Cholecystokinin-A and Peripheral Opioid Receptors in Rats" THE JOURNAL OF NUTRITION, [Online] vol. 132, September 2002 (2002-09), pages 2775-2780, XP002577477 Retrieved from the Internet: URL:http://jn.nutrition.org/cgi/content/fu ll/132/9/2775> [retrieved on 2010-04-12]**

**(Cont. next page)**

- TAKASHI NISHI, HIROSH HARA, TOHRU HIRA AND FUSAO TOMITA: "Dietary Protein Peptic Hydrolysates Stimulate Cholecystokinin Release via Direct Sensing by Rat Intestinal Mucosal Cells" EXPERIMENTAL BIOLOGY AND MEDECINE, [Online] vol. 226, no. 11, 2001, pages 1031-1036, XP002577478 Retrieved from the Internet: URL:http://ebm.rsmjournals.com/cgi/ content /abstract/226/11/1031> [retrieved on 2010-04-12]

**Description**

## CROSS REFERENCE TO RELATED APPLICATION

This application claims the priority of US provisional application number 61/141,931 filed December 31, 2008, which is hereby incorporated by reference in its entirety.

## FIELD OF THE INVENTION

The present invention generally relates to protein hydrolysates. In particular, the protein hydrolysates generally have enhanced cholecystokinin (CCK) releasing activity. The protein hydrolysates may be used to provide nutrients and to promote satiety.

## BACKGROUND OF THE INVENTION

The rates of obesity and the diseases associated with obesity are rising in the Unites States and throughout the world. While there is no single underlying cause, a contributing factor may be the fast-paced, harried life styles of many individuals and the concomitant consumption of fast food. Most fast food tends to be high in fat and/or sugar.

One viable target for combating the obesity epidemic may be cholecystokinin (CCK). CCK is a peptide hormone secreted into the circulation by intestinal cells in response to a protein or lipid rich meal. This peptide hormone mediates several physiological processes involved in the digestion of proteins and lipids. Secretion of CCK by the duodenal and intestinal mucosa is stimulated by fat- or protein-rich chyme entering the duodenum. CCK then induces satiety and reduced food intake through direct and/or indirect physiological and neural actions. Some direct actions include the inhibition of gastric emptying, inhibition of gastric acid secretion, and stimulation of gallbladder contraction. Combined with CCK's ability to stimulate neural pathways, CCK release produces a sense of "satiety," thus typically resulting in the consumption of fewer calories.

Cordier-Bussat et al (M. Peptones Stimulate Cholecystokinin Secretion and Gene Transcription in the Intestinal Cell Line STC-1, Endocrinology, vol. 138, n° 3, 1997, pages 1137-1144) describe the effects of peptones, which mainly consist of oligopeptides with a molecular weight below 1200, on CCK in STC-1 cells. These authors conclude that peptones specifically stimulate CCK secretion and that peptones dose-dependently increase the transcriptional activity of an 800-bp fragment on the CCK gene promoter transfected in STC-1 cells.

There is a need, therefore, for a nutritious, readily accessible food product that can be consumed "on the go." This food product should not only taste good, but it should also be nutritionally sound; that is, the product should be low in fat, high In protein, and high in vitamins and antioxidants. In addition, it would also be highly beneficial if the food product enhanced the release of CCK.

## SUMMARY OF THE INVENTION

Among the various aspects of the present invention is one aspect that encompasses a protein hydrolysate composition having cholecystokinin (CCK) releasing activity. The protein hydrolysate composition comprises a mixture of polypeptide fragments having an average size of less than about 100,000 Daltons, said mixture comprising a 10,000-100,000 Daltons polypeptide fraction, wherein a soluble fraction of the protein hydrolysate composition, at a concentration of at least about 0.5 mg/mL, stimulates CCK releasing activity with a potency substantially similar to greater than 50% of CCK released by STC-1 cells stimulated with 100 nM of phorbol 12-myristate-13-acetate (PMA) for 4 hours.

Another aspect of the invention provides a method for increasing the CCK releasing activity of a cell. The method comprises contacting the cell with a soluble fraction of a protein hydrolysate composition. The protein hydrolysate composition comprises a mixture of polypeptide fragments that have an average size of less than about 100,000 Daltons, said mixture comprising a 10,000-100,000 Daltons polypeptide fraction wherein, at a concentration of at least about 0.5 mg/mL, the protein hydrolysate composition stimulates CCK releasing activity with a potency substantially similar to greater than 50% of CCK released by STC-1 cells stimulated with 100 nM of PMA for 4 hours.

A further aspect of the present invention encompasses a method for promoting satiety in a subject. The method comprises administering to the subject an amount of a protein hydrolysate composition, wherein the amount administered results in a feeling of satiety by the subject. The protein hydrolysate composition comprises a mixture of polypeptide fragments having an average size of less than about 100,000 Daltons, said mixture comprising a 10,000-100,000 Daltons polypeptide fraction wherein, at a concentration of at least about 0.5 mg/mL, the protein hydrolysate composition stimulates CCK releasing activity with a potency substantially similar to greater than 50% of CCK released by STC-1 cells stimulated with 100 nM of PMA for 4 hours.

Still another aspect of the invention provides a food product comprising an edible material and a soluble fraction of a protein hydrolysate composition. The protein hydrolysate composition comprises a mixture of polypeptide fragments having an average size of less than about 100,000 Daltons, said mixture comprising a 10,000-100,000 Daltons polypeptide fraction wherein, at a concentration of at least about 0.5 mg/mL, the protein hydrolysate composition stimulates CCK releasing activity with a potency substantially similar to greater than 50% of CCK released by STC-1 cells stimulated with 100 nM of PMA for 4 hours.

## REFERENCE TO COLOR FIGURES.

The application contains at least one photograph executed in color. Copies of this patent application publication with color photographs will be provided by the Office upon request and payment of the necessary fee.

## DESCRIPTION OF THE FIGURES

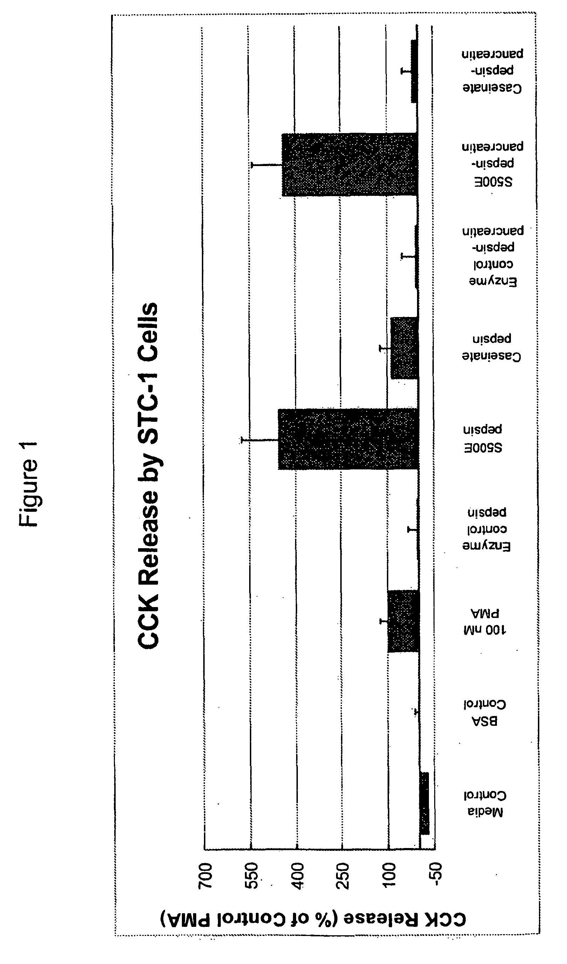

**Figure 1** shows the stimulation of CCK release by soy and caseinate protein hydrolysates prepared by digestion of each with pepsin or pepsin and pancreatin combined to mimic the digestion in the stomach and upper small intestine. The protein hydrolysates were added to the media of STC-1 cells at a protein concentration of approximately 2 mg/mL, and the enzyme controls were added at equivalent dilutions of the control reaction mixture (which included the enzyme (s) in the absence of protein substrate). Plotted is the % CCK released into the media of STC-1 cells stimulated by the different protein hydrolysates generated by the pepsin or pepsin and pancreatin enzymes, compared to the control 100 nM PMA (which was set as 100%). Cell culture media alone and 2 mg/mL bovine serum albumin (BSA) were used as negative background controls. The PMA control consisted of 100 nM PMA in cell culture media containing 2mg/mL BSA. %CCK released into the cell culture media was calculated as follows:

$$\% \text{ CCK release} = \frac{(\text{ng CCK}_{\text{sample well}} - \text{ng CCK}_{\text{BSA control well}})}{(\text{ng CCK}_{\text{PMA well}} - \text{ng CCK}_{\text{BSA control well}})} \times 100$$

Digestion of the intact soy protein with pepsin and pepsin-pancreatin yielded peptides with significantly more potent CCK releasing activity than the equivalent digestion of intact caseinate protein.

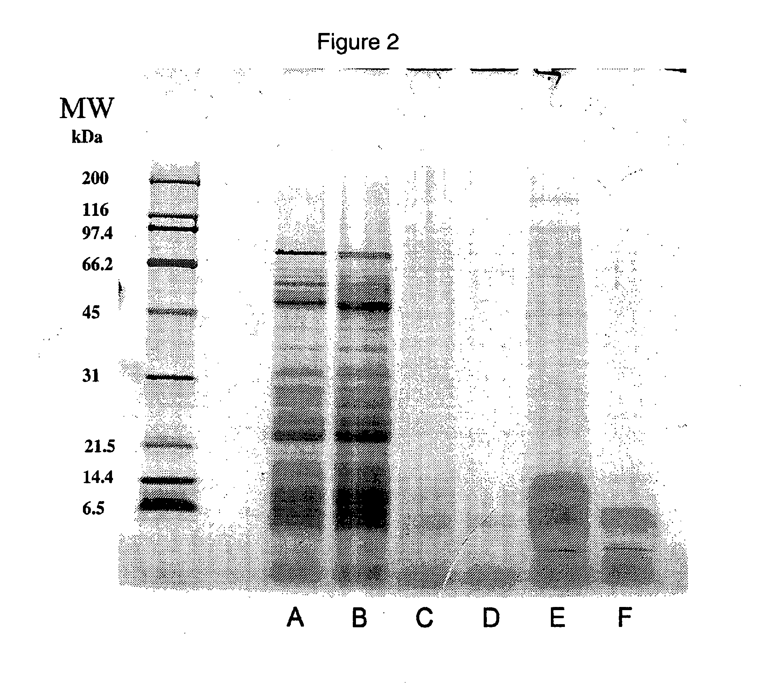

**Figure 2** presents an image of a Coomassie stained SDS polyacrylamide gel containing fractions from the tangential flow filtration of a potent CCK-releasing hydrolysate (SUPRO® 950/FXP950). This hydrolysate is generated by digestion with ALCALASE® from Novozymes((Bagsvaerd, Denmark) to a % degree of hydrolysis of approximately 9.6%. Lanes A, B, C, and D represent 10 μL samples of 1% (w/v) slurries of the unfractionated sample, the greater than100 kDa fraction, the 10-100 kDa fraction, and the less than 10 kDa fraction, respectively. Lanes E and F represent 10 μL samples of 5% (w/v) slurries of the 10-100 kDa fraction and the less than 10 kDa fraction, respectively. Sizes.of the molecular weight standards are indicated on the left.

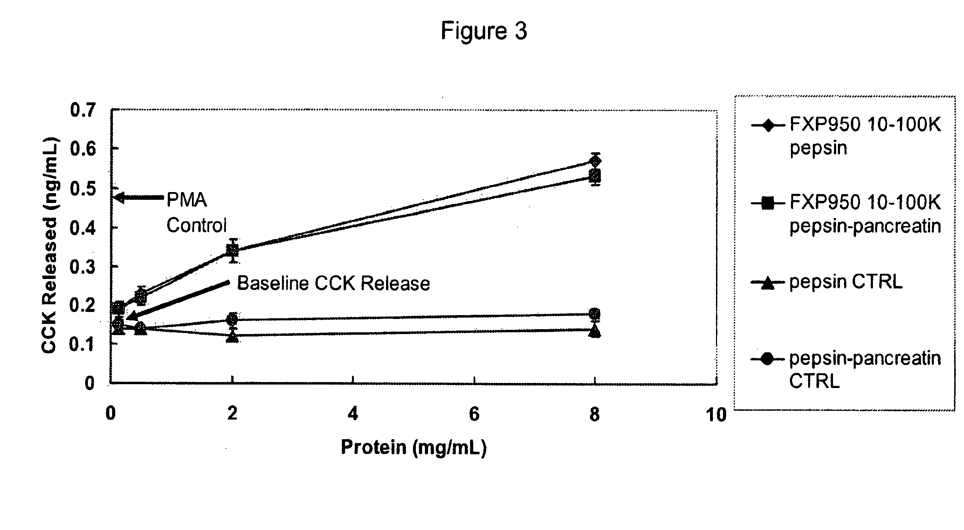

**Figure 3** shows the stimulation of CCK release by pepsin and pepsin-pancreatin digested preparations of the 10 -100 kDa fraction of SUPRO®950/FXP950, a hydrolyzed protein preparation described in Example 2.

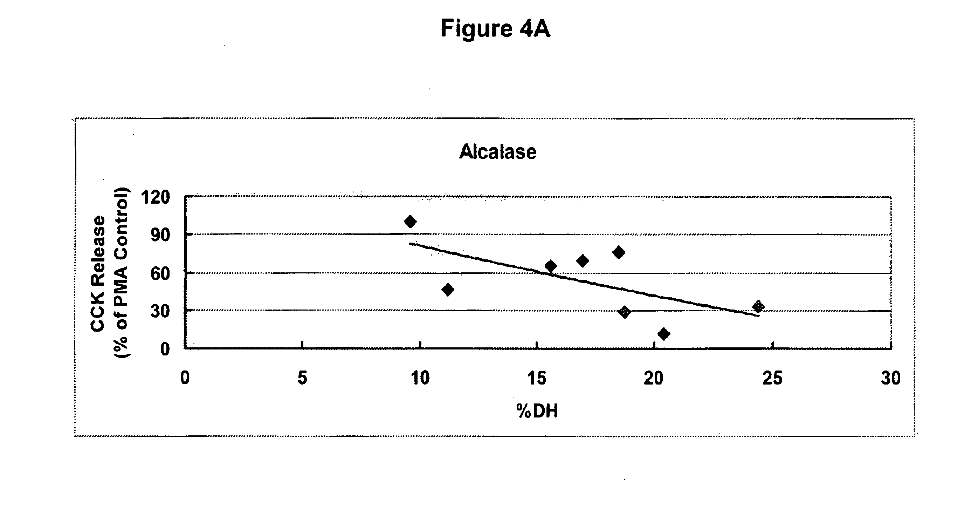

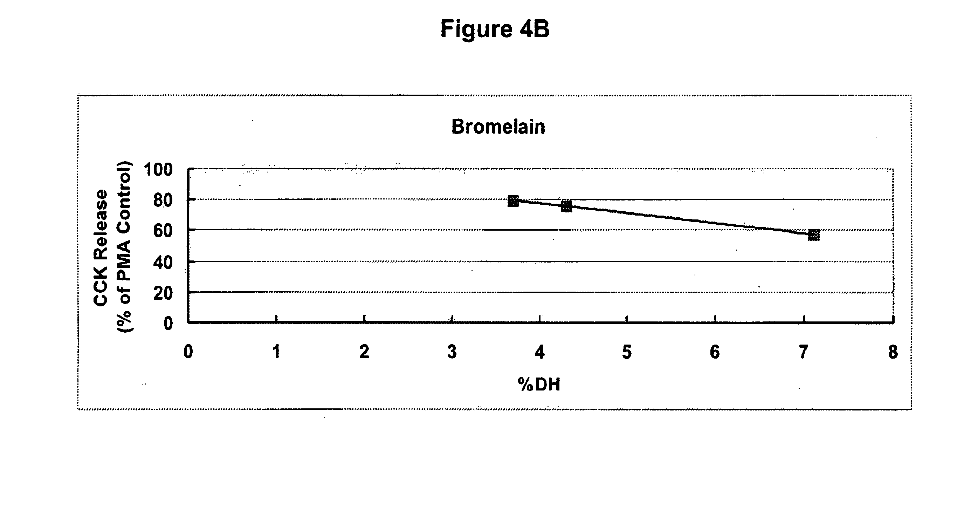

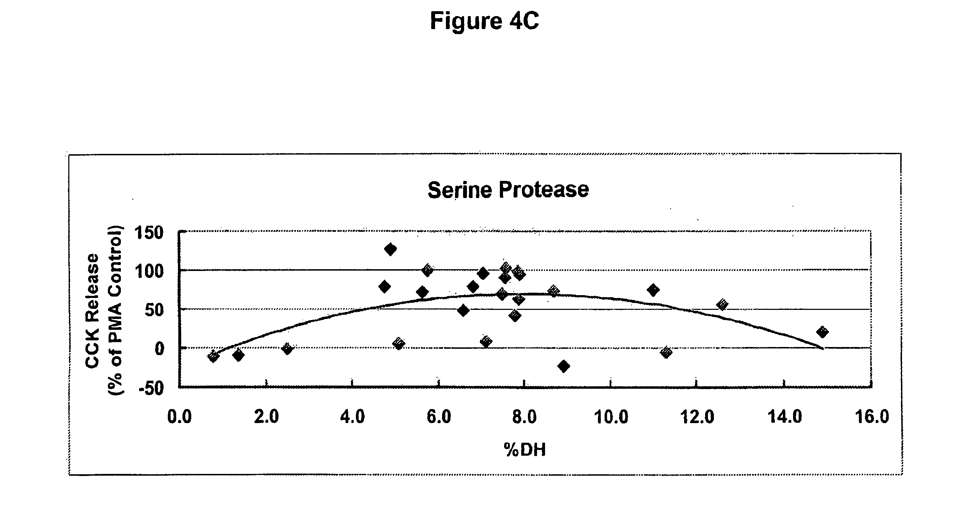

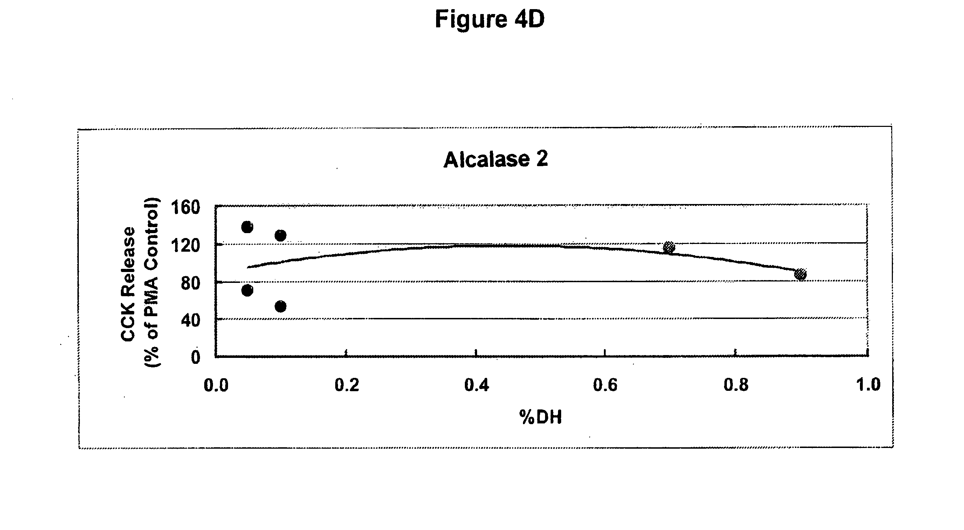

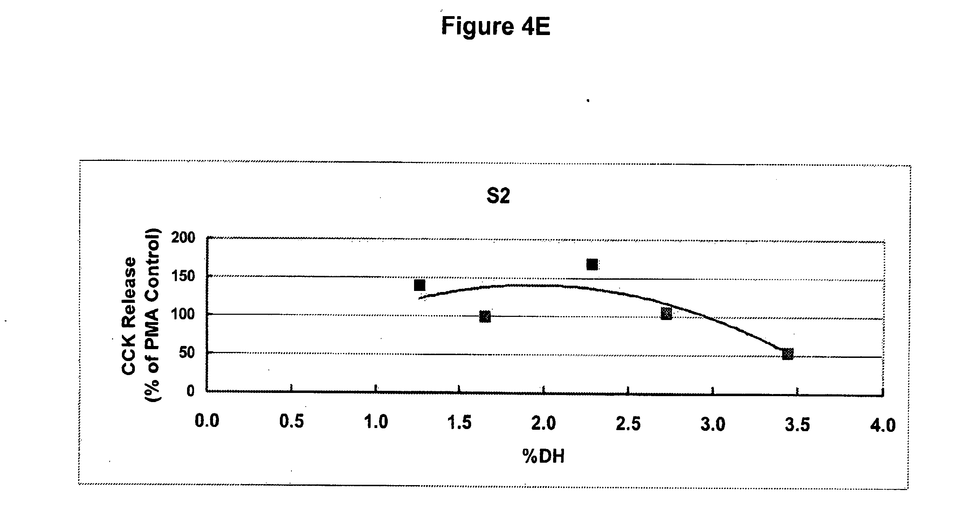

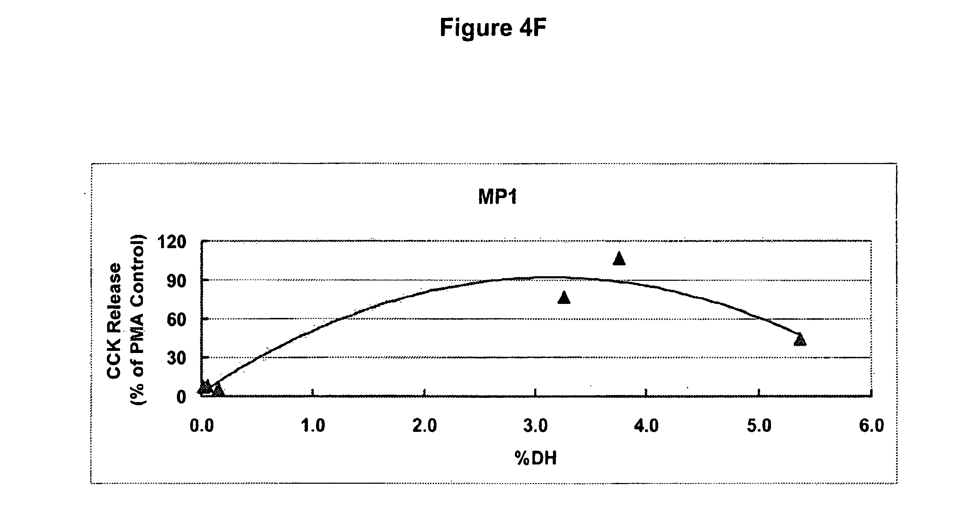

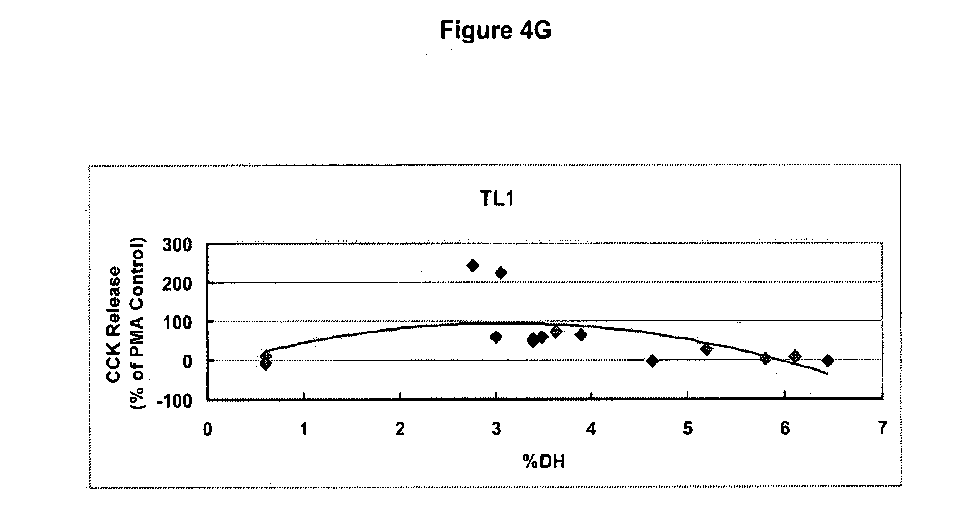

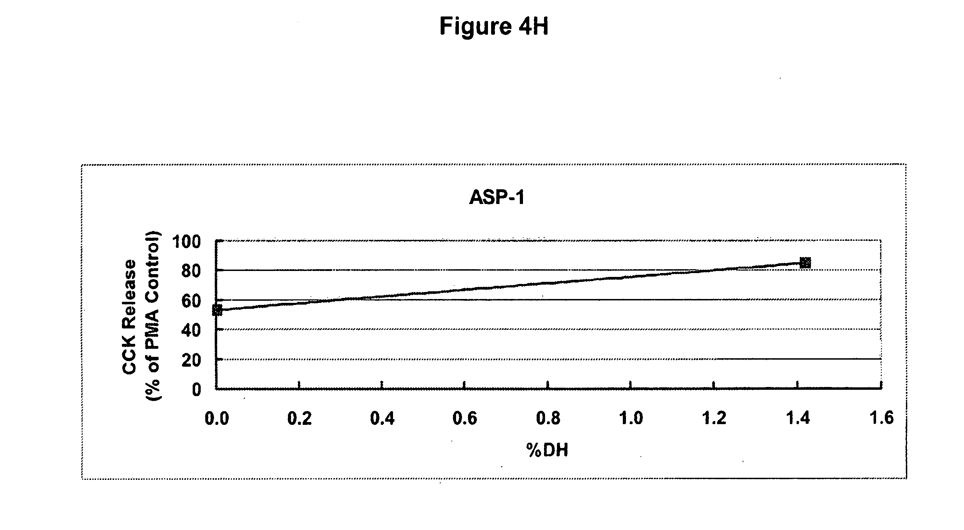

**Figures 4A-H** illustrate the stimulation of CCK release for different soy protein hydrolysate preparations. Plotted is the % CCK released into the media of STC-1 stimulated by the different soy protein hydrolysates generated with the different enzymes as indicated in Figures 4A-4H and with different % degrees of hydrolysis (%DH) obtained with the various incubation conditions compared to the % CCK released by 100 nM PMA, which was set at 100%. %CCK released into the cell culture media is calculated as described for **Figure 1.** PMA induces CCK through a direct stimulation of protein kinase C (PKC). Each soy protein hydrolysate was added to STC-1 cells at a final protein concentration of approximately 2 mg/mL. **Figure 4A** shows stimulation of CCK release for soy protein hydrolysates treated with Alcalase. **Figure 4B** shows stimulation of CCK release for soy protein hydrolysates treated with Bromelain. **Figure 4C** shows stimulation of CCK release for soy protein hydrolysates treated with serine protease from *Nocardiopsis prasina.* **Figure 4D** shows stimulation of CCK release for soy protein hydrolysates treated with ALCALASE® 2. **Figure 4E** shows stimulation of CCK release for soy protein hydrolysates treated with S2. **Figure 4F** shows stimulation of CCK release for soy protein hydrolysates treated with MP1. **Figure 4G** shows stimulation of CCK release for soy protein hydrolysates treated with TL1. **Figure 4H** shows stimulation of CCK release for soy protein hydrolysates treated with ASP-1.

## DETAILED DESCRIPTION OF THE INVENTION

It is known that protein, in general, stimulates the release of CCK by enteroendocrine cells in the intestinal track of animals, including humans (Liddle, R.A., et al., (1986) Proteins But Not Amino Acids, Carbohydrates, or Fats Stimulate Cholecystokinin Secretion in the Rat. Am. J. Physiol. 251 (Gastrointest. Liver Physiol. 14): G243-G248). Since protein that passes into the intestine experiences digestion by enzymes such as pepsin and a mixture of digestive enzymes from the pancreas (pancreatin), we show in **Figure 1** that an intact soy protein digested by these enzymes yields peptides that have CCK releasing activity on enteroendocrine cells. **Figure 1** also shows that sodium caseinate treated with these same enzymes yields peptides with less potent CCK releasing activity compared to those generated after digestion of the intact soy protein. The method for digestion of the soy protein and caseinate, used to generate the data in **Figure 1,** which mimic the in vivo stomach and upper intestinal digestion, is a modification of the previously published procedures of Schasteen (Schasteen, C.S., et al., (2007) Correlation of an Immobilized Digestive Enzyme Assay With Poultry True Amino Acid Digestibility for Soybean Meal. Poultry Science 86(2), 343-348) and Higaki ([Higaki, N., et al., (2006) Biosci. Biotechnol. Biochem. 70(12), 2844-2852). Protein samples were solubilized in 20 volumes of 0.01 M HCl and digested by pepsin (Sigma-Aldrich #P7012) at an enzyme-substrate ratio of 1:200 (w/w), pH 2.3 and 37°C for 4 hours. After the pepsin digestion, 2.5 M NaOH was added to the mixture to adjust the pH to 8.0, and pancreatin (Sigma-Aldrich #P3292) was added at a ratio of 1:200 (w/w) and digestion was continued for another 4 to 18 hours. Degree of hydrolysis was determined by the reaction of primary amine groups with o-phthalaldehyde (OPA) vs. total amount of primary amine present in sample after acid hydrolysis (110°C for 24 hours) (known as the "OPA method"). The protein hydrolysates were added to the media of STC-1 cells at a protein concentration of 2 mg/mL, and the enzyme controls were added at equivalent dilutions of the control reaction mixture (which included the enzyme(s) in the absence of protein substrate). Plotted is the % CCK released into the media of STC-1 cells stimulated by the pepsin and pepsin-pancreatin treated protein samples compared to the amount of CCK released by the positive control 100 nM PMA (which was set at 100%). %CCK released into the cell culture media is calculated as follows:

$$\% \text{ CCK release} = \frac{(\text{ng CCK}_{\text{sample well}} - \text{ng CCK}_{\text{BSA control well}})}{(\text{ng CCK}_{\text{PMA well}} - \text{ng CCK}_{\text{BSA control well}})} \times 100$$

It has been discovered, as illustrated in the examples, that enzymatic digestion of a protein into polypeptide fragments that have an average size of less than about 100,000 Daltons by enzymes that can be used in commercial ingredient processing results in compositions having enhanced CCK releasing activity. Because CCK promotes satiety through the central nervous system, and also slows gastric emptying, the protein hydrolysate compositions may be included in a variety of food products to both promote satiety and provide nutrients.

### (I)Process for Preparing a Protein Hydrolysate

One aspect of the present invention provides a process for preparing a protein hydrolysate comprising a mixture of polypeptide fragments having an average size of less than 100,000 Daltons, said mixture comprising a 10,000-100,000 Daltons polypeptide fraction. The process comprises contacting a protein material with one or more enzymes that cleaves the protein material into polypeptide fragments of the desired size. Reactants and reaction parameters are described more fully below.

### (a) protein material

Non-limiting examples of suitable protein materials include plant proteins, such as soy or non-soy proteins (e.g., barley, canola, lupin, maize, oat, pea, potato, rice, wheat, etc.) and animal proteins, such as egg proteins, gelatin, and the like.

in some embodiments, the protein material may be derived from soy. A variety of soy protein materials may be used in the process of the invention to generate a soy protein hydrolysate. In general, the soy protein material may be derived from whole soybeans in accordance with methods known in the art. The whole soybeans may be standard soybeans (i.e., non genetically modified soybeans), genetically modified soybeans (such as, e.g., soybeans with modified oils, soybeans with modified carbohydrates, soybeans with modified protein subunits, and so forth) or combinations thereof. Suitable examples of soy protein material include soy extract, soymilk, soymilk powder, soy curd, soy flour, soy protein isolate, soy protein concentrate, soy whey protein, and mixtures thereof.

In one embodiment, the soy protein material used in the process may be a soy protein isolate (also called isolated soy protein, or ISP). In general, soy protein isolates have a protein content of at least about 90% soy protein on a moisture-free basis. The soy protein isolate may comprise intact soy proteins or it may comprise partially hydrolyzed soy proteins. The soy protein isolate may have a high content of various subunits such as 7S, 11S, 2S, etc. Non-limiting examples of soy protein isolates that may be used in the present invention are commercially available, for example, from Solae, LLC (St. Louis, MO), and include SUPRO® 500E, SUPRO® 620, SUPRO® 760, SUPRO® 670, SUPRO® 710, SUPRO® EX 33, SUPRO® 313.

In another embodiment, the soy protein material may be a soy protein concentrate, which has a protein content of about 65% to less than about 90% on a moisture-free basis. Examples of suitable soy protein concentrates useful in the invention include ALPHA® DSP-C, Proton™, ALPHA® 12 and ALPHA® 5800, which are commercially available from Solae, LLC. Alternatively, soy protein concentrate may be blended with the soy protein isolate to substitute for a portion of the soy protein isolate as a source of soy protein material.

In yet another embodiment, the soy protein material may be soy flour, which has a protein content of about 49% to about 65% on a moisture-free basis. The soy flour may be defatted soy flour, partially defatted soy flour, or full fat soy flour. The soy flour may be blended with soy protein isolate or soy protein concentrate.

When soy flour is used, the starting material is typically defatted soy flour or flakes. Full fat soybeans contain approximately 40% protein by weight and approximately 20% oil by weight. These whole full fat soybeans may be defatted through conventional processes when a defatted soy flour or flakes form the starting protein material. For example, the soybean may be cleaned, dehulled, cracked, passed through a series of flaking rolls and then subjected to solvent extraction by use of hexane or other appropriate solvents to extract the oil and produce "spent flakes". The defatted flakes may be ground to produce a soy flour. Although the process is yet to be employed with full fat soy flour, it is believed that full fat soy flour may also serve as a protein source. However, where full fat soy flour is processed, it is most likely necessary to use a separation step, such as three stage centrifugation to remove oil.

In another alternate embodiment, the soy protein material may be soy storage protein that has been separated into major fractions (15S, 11S, 7S, and 2S) on the basis of sedimentation in a centrifuge. In general, the 11S fraction is highly enriched in glycinins, and the 7S fraction is highly enriched in beta-conglycinins.

In another embodiment, the protein material may be derived from a plant other than soy. By way of non-limiting example, suitable plants include amaranth, arrowroot, barley, buckwheat, canola, cassava, channa (garbanzo), legumes, lentils, lupin, maize, millet, oat, pea, potato, rice, rye, sorghum, sunflower, tapioca, triticale, wheat, and mixtures thereof. Especially preferred plant proteins include barley, canola, lupin, maize, oat, pea, potato, rice, wheat, and combinations thereof. In one embodiment, the plant protein material may be canola meal, canola protein isolate, canola protein concentrate, or combinations thereof. In another embodiment, the plant protein material may be maize or corn protein powder, maize or corn protein concentrate, maize or corn protein isolate, maize or corn germ, maize or corn gluten, maize or corn gluten meal, maize or corn flour, zein protein, or combinations thereof. In still another embodiment, the plant protein material may be barley powder, barley protein concentrate, barley protein isolate, barley meal, barley flour, or combinations thereof. In an alternate embodiment, the plant protein material may be lupin flour, lupin protein isolate, lupin protein concentrate, or combinations thereof. In another alternate embodiment, the plant protein material may be oatmeal, oat flour, oat protein flour, oat protein isolate, oat protein concentrate, or combinations thereof. In yet another embodiment, the plant protein material may be pea flour, pea protein isolate, pea protein concentrate, or combinations thereof. In still another embodiment, the plant protein material may be potato protein powder, potato protein isolate, potato protein concentrate, potato flour, or combinations thereof. In a further embodiment, the plant protein material may be rice flour, rice meal, rice protein powder, rice protein isolate, rice protein concentrate, or combinations thereof. In another alternate embodiment, the plant protein material may be wheat protein powder, wheat gluten, wheat germ, wheat flour, wheat protein isolate, wheat protein concentrate, solubilized wheat proteins, or combinations thereof.

In other embodiments, the protein material may be derived from an animal source. In one embodiment, the animal protein material may be derived from eggs. Non-limiting examples of suitable egg proteins include powdered egg, dried egg solids, dried egg white protein, liquid egg white protein, egg white protein powder, isolated ovalbumin protein, and combinations thereof. Egg proteins may be derived from the eggs of chicken, duck, goose, quail, or other birds. In an alternate embodiment, the protein material may be derived from a dairy source. Suitable dairy proteins include non-fat dry milk powder, milk protein isolate, milk protein concentrate, acid casein, caseinate (e.g., sodium caseinate, calcium caseinate, and the like), whey protein isolate, whey protein concentrate, and combinations thereof. The milk protein material may be derived from cows, goats, sheep, donkeys, camels, camelids, yaks, water buffalos, etc. In a further embodiment, the protein may be derived from the muscles, organs, connective tissues, or skeletons of land-based or aquatic animals. As an example, the animal protein may be gelatin, which is produced by partial hydrolysis of collagen extracted from the bones, connective tissues, organs, etc, from cattle or other animals.

It is also envisioned that combinations of a soy protein material and at least one other protein material also may be used in the process of the invention. That is, a protein hydrolysate composition may be prepared from a combination of a soy protein material and at least one other protein material. In one embodiment, a protein hydrolysate composition may be prepared from a combination of a soy protein material and one other protein material selected from the group consisting of barley, canola, lupin, maize, oat, pea, potato, rice, wheat, animal material, dairy, and egg. In another embodiment, a protein hydrolysate composition may be prepared from a combination of a soy protein material and two other protein materials selected from the group consisting of barley, canola, lupin, maize, oat, pea, potato, rice, wheat, animal material, dairy, and egg. In further embodiments, a protein hydrolysate composition may be prepared from a combination of a soy protein material and three or more other protein materials selected from the group consisting of barley, canola, lupin, maize, oat, pea, potato, rice, wheat, animal material, dairy, and egg.

The concentrations of the soy protein material and the other protein material used in combination can and will vary. The amount of soy protein material may range from about 1% to about 99% of the total protein used in the combination. In one embodiment, the amount of soy protein material may range from about 1% to about 20% of the total protein used in combination. In another embodiment, the amount of soy protein material may range from about 20% to about 40% of the total protein used in combination. In still another embodiment, the amount of soy protein material may range from about 40% to about 80% of the total protein used in combination. In a further embodiment, the amount of soy protein material may range from about 80% to about 99% of the total protein used in combination. Likewise, the amount of the (at least one) other protein material may range from about 1% to about 99% of the total protein used in combination. In one embodiment, the amount of other protein material may range from about 1% to about 20% of the total protein used in combination. In another embodiment, the amount of other protein material may range from about 20% to about 40% of the total protein used in combination. In still another embodiment, the amount of other protein material may range from about 40% to about 80% of the total protein used in combination. In a further embodiment, the amount of other protein material may range from about 80% to about 99% of the total protein used in combination.

### (b) protein slurry

In the process of the invention, the protein material is typically mixed or dispersed in water to form a slurry comprising about 1% to about 40% protein by weight (on an "as is" basis). In one embodiment, the slurry may comprise about 1% to about 5% protein (as is) by weight. In another embodiment, the slurry may comprise about 6% to about 10% protein (as is) by weight. In a further embodiment, the slurry may comprise about 11 % to about 15% protein (as is) by weight. In still another embodiment, the slurry may comprise about 16% to about 20% protein (as is) by weight. In yet another embodiment, the slurry may comprise about 21% to about 40% protein (as is) by weight. The water may include food grade dispersants such as ethanol, glycerol, and the like.

After the protein material is dispersed in water, the slurry of protein material may be heated from about 70°C to about 90°C for about 2 minutes to about 20 minutes to inactivate putative endogenous protease inhibitors. Typically, the pH and the temperature of the protein slurry are adjusted so as to optimize the hydrolysis reaction, and in particular, to ensure the digestion enzyme used in the hydrolysis reaction functions near its optimal activity level. The pH of the protein slurry may be adjusted and monitored according to methods generally known in the art. The pH of the protein slurry may be adjusted and maintained at from about 3.0 to about 11.0. In other embodiments, the pH of the protein slurry may be adjusted and maintained at from about 3.0 to about 4.0, about 5.0 to about 6.0, and about 7.0 to about 8.0. In another embodiment, the pH of the protein slurry may be adjusted and maintained at from about 8.0 to about 9.0. In an alternate embodiment, the pH of the protein slurry may be adjusted and maintained at from about 9.0 to about 10.0, and about 10.0 to about 11.0. The temperature of the protein slurry can be adjusted and maintained at from about 25°C to about 80°C during the hydrolysis reaction in accordance with methods known in the art.

### (c) enzyme digestion

The hydrolysis reaction is generally initiated by adding an enzyme or a combination of enzymes to the slurry of protein material. Typically, the enzyme may be a food-grade enzyme having optimal activity at a pH from about 3.0 to about 11.0 and at a temperature from about 25°C to about 80°C. The enzyme may be of plant, animal, or microbial origin.

The enzyme will typically be an endopeptidase. Endopeptidases act preferentially in the inner regions of peptide chains away from the N and C termini. Several endopeptidases are suitable for use in the process of the invention. In one embodiment, the endopeptidase may be serine protease from *Nocardiopsis prasina* (SEQ ID NO: 2 in International Application No. WO 2005035747, which is incorporated by reference in its entirety). In another embodiment, the endopeptidase may be subtilisin protease from *Bacillus licheniformis,* which is available as ALCALASE® from Novozymes (Bagsvaerd, Denmark). In yet another embodiment, the endopeptidase may be serine protease also called glutamyl endopeptidase (termed "GE") from *Bacillus licheniformis* (UNIPROT: P80057 as disclosed and characterized in US Patent Nos. 4,266,031, 5,874,278, and 5,459,064 and International Application Nos. WO 01/16285, WO 92/13964, WO 91/13553, and WO 91/13554, each of which is incorporated by reference in its entirety). In still another embodiment, the endopeptidase may be trypsin-like protease (termed "TL1") from *Fusarium oxysporum* (SWISSPROT No. P35049) (US Patent Nos. 5,288,627 and 5,693,520 each of which is hereby incorporated by reference in its entirety). In an alternate embodiment, the endopeptidase may be lysyl endopeptidase (termed "LE") from *Achromobacter lyticus* (UNIPROT:P15636). In a further embodiment, the endopeptidase may be a more purified form of subtilisin protease from *Bacillus licheniformis* (termed "Alcalase® 2") In still other embodiments, the endopeptidase may be a trypsin-like protease from *Fusarium solani* (GENESEQP:ADZ80577). Suitable enzymes further include subtilisin protease 2 (S2), metallo protease 1 (MP1), and aspartate protease 1 (ASP-1). Other suitable enzymes include bromelain, subtilisin, chymotrypsin, trypsin, pepsin, and elastase. In some embodiments, a combination of endopeptidases may be used.

In a further embodiment, the endopeptidase may be combined with at least one exopeptidase. Generally, exopeptidases act only near the ends of polypeptide chains at the N or C terminus. Those acting at a free N terminus liberate a single amino acid residue (i.e., aminopeptidases), a dipeptide (i.e., dipeptidyl-peptidases) or a tripeptide (i.e., tripeptidyl-peptidases). The exopeptidases acting at a free C terminus liberate a single amino acid (i.e., carboxypeptidases) or a dipeptide (i.e., peptidyldipeptidases). Some exopeptidases are specific for dipeptides (i.e., dipeptidases) or remove terminal residues that are substituted, cyclized or linked by isopeptide bonds. Isopeptide bonds are peptide linkages other than those of a carboxyl group to an $\alpha$-amino group, and this group of enzymes is characterized by omega peptidases.

Non-limiting examples of exopeptidases suitable for use in the process of the invention include carboxypeptidase D from *Aspergillus oryzae* (UNIPROT:Q2TZ11), carboxypeptidase Y from *Aspergillus oryzae* (UNIPROT:Q2TYA1), aminopeptidase from *Aspergillus* oryzae (International Application No. WO 96/28542, which is incorporated by reference in its entirety), and aminopeptidase from *Bacillus licheniformis* (UNIPROT:Q65DH7).

The amount of enzyme added to the protein material can and will vary, depending upon the desired degree of hydrolysis and the duration of the hydrolysis reaction. The amount may range from about 1 mg to about 5000 mg of enzyme protein per kilogram of protein material. In another embodiment, the amount may range from 10 mg to about 2000 mg of enzyme protein per kilogram of protein material. In yet another embodiment, the amount may range from about 50 mg to about 1000 mg of enzyme protein per kilogram of protein material.

As will be appreciated by a skilled artisan, the duration of the hydrolysis reaction can and will vary depending upon the enzyme, the protein material, and the desired degree of hydrolysis. Generally speaking, the duration of the hydrolysis reaction may range from a few minutes to many hours, such as, from about 30 minutes to about 48 hours. To end the hydrolysis reaction, the composition may be heated to a temperature that is high enough to inactivate the enzyme. For example, heating the composition to a temperature of approximately 90°C will substantially heat-inactivate most enzymes. Other methods of inactivation include cooling below 10°C and/or lowering pH below about 3.0, depending on the enzyme used.

Various combinations of protein material and enzyme are presented in Table A. **Table A.** Preferred Combinations.

| Protein Material | Endopeptidase |
|---|---|
| Soy | Serine protease |
| Soy | ALCALASE® |
| Soy | GE |
| Soy | TL1 |
| Soy | LE |
| Soy | Bromelain |
| Soy | Alcalase® 2 |
| Soy | S2 |
| Soy | MP1 |
| Soy | ASP-1 |
| Barley | Serine protease |
| Barley | ALCALASE® |
| Barley | GE |
| Barley | TL1 |
| Barley | LE |
| Barley | Bromelain |
| Barley | Alcalase® 2 |
| Barley | S2 |
| Barley | MP1 |
| Barley | ASP-1 |
| Canola | Serine protease |

(continued)

| Protein Material | Endopeptidase |
|---|---|
| Canola | ALCALASE® |
| Canola | GE |
| Canola | TL1 |
| Canola | LE |
| Canola | Bromelain |
| Canola | Alcalase® 2 |
| Canola | S2 |
| Canola | MP1 |
| Canola | ASP-1 |
| Lupin | Serine protease |
| Lupin | ALCALASE® |
| Lupin | GE |
| Lupin | TL1 |
| Lupin | LE |
| Lupin | Bromelain |
| Lupin | Alcalase® 2 |
| Lupin | S2 |
| Lupin | MP1 |
| Lupin | ASP-1 |
| Maize | Serine protease |
| Maize | ALCALASE® |
| Maize | GE |
| Maize | TL1 |
| Maize | LE |
| Maize | Bromelain |
| Maize | Alcalase®2 |
| Maize | S2 |
| Maize | MP1 |
| Maize | ASP-1 |
| Oat | Serine protease |
| Oat | ALCALASE® |
| Oat | GE |
| Oat | TL1 |
| Oat | LE |
| Oat | Bromelain |
| Oat | Alcalase® 2 |
| Oat | S2 |
| Oat | MP1 |

(continued)

| Protein Material | Endopeptidase |
| --- | --- |
| Oat | ASP-1 |
| Pea | Serine protease |
| Pea | ALCALASE® |
| Pea | GE |
| Pea | TL1 |
| Pea | LE |
| Pea | Bromelain |
| Pea | Alcalase® 2 |
| Pea | S2 |
| Pea | MP1 |
| Pea | ASP-1 |
| Potato | Serine protease |
| Potato | ALCALASE® |
| Potato | GE |
| Potato | TL1 |
| Potato | LE |
| Potato | Bromelain |
| Potato | Alcalase® 2 |
| Potato | S2 |
| Potato | MP1 |
| Potato | ASP-1 |
| Rice | Serine protease |
| Rice | ALCALASE® |
| Rice | GE |
| Rice | TL1 |
| Rice | LE |
| Rice | Bromelain |
| Rice | Alcalase® 2 |
| Rice | S2 |
| Rice | MP1 |
| Rice | ASP-1 |
| Wheat | Serine protease |
| Wheat | ALCALASE® |
| Wheat | GE |
| Wheat | TL1 |
| Wheat | LE |
| Wheat | Bromelain |
| Wheat | Alcalase® 2 |

(continued)

| Protein Material | Endopeptidase |
|---|---|
| Wheat | S2 |
| Wheat | MP1 |
| Wheat | ASP-1 |
| Egg | Serine protease |
| Egg | ALCALASE® |
| Egg | GE |
| Egg | TL1 |
| Egg | LE |
| Egg | Bromelain |
| Egg | Alcalase® 2 |
| Egg | S2 |
| Egg | MP1 |
| Egg | ASP-1 |
| Dairy | Serine protease |
| Dairy | ALCALASE® |
| Dairy | GE |
| Dairy | TL1 |
| Dairy | LE |
| Dairy | Bromelain |
| Dairy | Alcalase® 2 |
| Dairy | S2 |
| Dairy | MP1 |
| Dairy | ASP-1 |
| Animal | Serine protease |
| Animal | ALCALASE® |
| Animal | GE |
| Animal | TL1 |
| Animal | LE |
| Animal | Bromelain |
| Animal | Alcalase® 2 |
| Animal | S2 |
| Animal | MP1 |
| Animal | ASP-1 |
| Soy and Barley | Serine protease |
| Soy and Barley | ALCALASE® |
| Soy and Barley | GE |
| Soy and Barley | TL1 |
| Soy and Barley | LE |

(continued)

| Protein Material | Endopeptidase |
| --- | --- |
| Soy and Barley | Bromelain |
| Soy and Barley | Alcalase® 2 |
| Soy and Barley | S2 |
| Soy and Barley | MP1 |
| Soy and Barley | ASP-1 |
| Soy and Canola | Serine protease |
| Soy and Canola | ALCALASE® |
| Soy and Canola | GE |
| Soy and Canola | TL1 |
| Soy and Canola | LE |
| Soy and Canola | Bromelain |
| Soy and Canola | Alcalase® 2 |
| Soy and Canola | S2 |
| Soy and Canola | MP1 |
| Soy and Canola | ASP-1 |
| Soy and Lupin | Serine protease |
| Soy and Lupin | ALCALASE® |
| Soy and Lupin | GE |
| Soy and Lupin | TL1 |
| Soy and Lupin | LE |
| Soy and Lupin | Bromelain |
| Soy and Lupin | Alcalase® 2 |
| Soy and Lupin | S2 |
| Soy and Lupin | MP1 |
| Soy and Lupin | ASP-1 |
| Soy and Maize | Serine protease |
| Soy and Maize | ALCALASE® |
| Soy and Maize | GE |
| Soy and Maize | TL1 |
| Soy and Maize | LE |
| Soy and Maize | Bromelain |
| Soy and Maize | Alcalase® 2 |
| Soy and Maize | S2 |
| Soy and Maize | MP1 |
| Soy and Maize | ASP-1 |
| Soy and Oat | Serine protease |
| Soy and Oat | ALCALASE® |
| Soy and Oat | GE |

(continued)

| Protein Material | Endopeptidase |
| --- | --- |
| Soy and Oat | TL1 |
| Soy and Oat | LE |
| Soy and Oat | Bromelain |
| Soy and Oat | Alcalase® 2 |
| Soy and Oat | S2 |
| Soy and Oat | MP1 |
| Soy and Oat | ASP-1 |
| Soy and Pea | Serine protease |
| Soy and Pea | ALCALASE® |
| Soy and Pea | GE |
| Soy and Pea | TL1 |
| Soy and Pea | LE |
| Soy and Pea | Bromelain |
| Soy and Pea | Alcalase® 2 |
| Soy and Pea | S2 |
| Soy and Pea | MP1 |
| Soy and Pea | ASP-1 |
| Soy and Potato | Serine protease |
| Soy and Potato | ALCALASE® |
| Soy and Potato | GE |
| Soy and Potato | TL1 |
| Soy and Potato | LE |
| Soy and Potato | Bromelain |
| Soy and Potato | Alcalase® 2 |
| Soy and Potato | S2 |
| Soy and Potato | MP1 |
| Soy and Potato | ASP-1 |
| Soy and Rice | Serine protease |
| Soy and Rice | ALCALASE® |
| Soy and Rice | GE |
| Soy and Rice | TL1 |
| Soy and Rice | LE |
| Soy and Rice | Bromelain |
| Soy and Rice | Alcalase® 2 |
| Soy and Rice | S2 |
| Soy and Rice | MP1 |
| Soy and Rice | ASP-1 |
| Soy and Wheat | Serine protease |

(continued)

| Protein Material | Endopeptidase |
|---|---|
| Soy and Wheat | ALCALASE® |
| Soy and Wheat | GE |
| Soy and Wheat | TL1 |
| Soy and Wheat | LE |
| Soy and Wheat | Bromelain |
| Soy and Wheat | Alcalase® 2 |
| Soy and Wheat | S2 |
| Soy and Wheat | MP1 |
| Soy and Wheat | ASP-1 |
| Soy and Egg | Serine protease |
| Soy and Egg | ALCALASE® |
| Soy and Egg | GE |
| Soy and Egg | TL1 |
| Soy and Egg | LE |
| Soy and Egg | Bromelain |
| Soy and Egg | Alcalase® 2 |
| Soy and Egg | S2 |
| Soy and Egg | MP1 |
| Soy and Egg | ASP-1 |
| Soy and Dairy | Serine protease |
| Soy and Dairy | ALCALASE® |
| Soy and Dairy | GE |
| Soy and Dairy | TL1 |
| Soy and Dairy | LE |
| Soy and Dairy | Bromelain |
| Soy and Dairy | Alcalase® 2 |
| Soy and Dairy | S2 |
| Soy and Dairy | MP1 |
| Soy and Dairy | ASP-1 |
| Soy and Animal | Serine protease |
| Soy and Animal | ALCALASE® |
| Soy and Animal | GE |
| Soy and Animal | TL1 |
| Soy and Animal | LE |
| Soy and Animal | Bromelain |
| Soy and Animal | Alcalase® 2 |
| Soy and Animal | S2 |
| Soy and Animal | MP1 |

(continued)

| Protein Material | Endopeptidase |
|---|---|
| Soy and Animal | ASP-1 |

### (II) Protein Hydrolysate

The protein hydrolysate composition generally enhances CCK release and thereby, promotes satiety when consumed. As illustrated in the examples, a concentration of at least about 0.5 mg/mL of the soy protein hydrolysate composition stimulates CCK releasing activity. The CCK stimulating effect of optimal hydrolysates added at between 0.5 and 8 mg/mL of protein is similar to greater than 50% of CCK released by STC-1 cells stimulated with 100 nM of PMA for 4 hours.

In one embodiment, the soluble fraction of a protein hydrolysate composition may stimulate CCK releasing activity from about 50% to about 100% of CCK released by STC-1 cells stimulated with 100 nM of PMA for 4 hours. In another embodiment, the soluble fraction of a protein hydrolysate composition may stimulate CCK releasing activity from about 100% to about 200% of CCK released by STC-1 cells stimulated with 100 nM of PMA for 4 hours. In a further embodiment, the soluble fraction of a protein hydrolysate composition may stimulate CCK releasing activity from about 200% to about 300% of CCK released by STC-1 cells stimulated with 100 nM of PMA for 4 hours. In yet another embodiment, the soluble fraction of a protein hydrolysate composition may stimulate CCK releasing activity from about 300% to about 500% of CCK released by STC-1 cells stimulated with 100 nM of PMA for 4 hours. In yet another embodiment, the soluble fraction of a protein hydrolysate composition may stimulate CCK releasing activity from about 500% to about 1000% of CCK released by STC-1 cells stimulated with 100nM PMA for 4 hours.

The degree of hydrolysis of the protein hydrolysate composition can and will vary depending upon the source of the protein material, the protease(s) used, and the conditions of the hydrolysis reaction. The degree of hydrolysis (DH) refers to the percentage of peptide bonds cleaved versus the starting number of peptide bonds. For example, if a starting protein containing five hundred peptide bonds is hydrolyzed until fifty of the peptide bonds are cleaved, then the DH of the resulting hydrolysate is 10%. The degree of hydrolysis may be determined using the trinitrobenzene sulfonic (TNBS), the colorimetric method or the ortho-phthaldialdehye (OPA) method, which are known to those skilled in the art. The higher the degree of hydrolysis the greater the extent of protein hydrolysis. Typically, as the protein is further hydrolyzed (i.e., the higher the DH), the molecular weight of the peptide fragments decreases, the peptide profile changes accordingly. The DH may be measured in the entire hydrolysate (i.e., whole fraction) or the DH may be measured in the soluble fraction of the hydrolysate (i.e., the supernatant fraction after centrifugation of the hydrolysate at about 500 to about 1000 x g for about 5 to about 10 minutes).

Typically, each of the protein hydrolysate compositions of the invention will have a degree of hydrolysis that ranges from about 0.05% to about 35%. In one embodiment, the degree of hydrolysis of the protein hydrolysate composition may range from about 0.05% to about 1%. In another embodiment, the degree of hydrolysis of the protein hydrolysate composition may range from about 1% to about 10%. In a further embodiment, the degree of hydrolysis of the protein hydrolysate composition may range from about 10% to about 20%. In still another embodiment, the degree of hydrolysis of the protein hydrolysate composition may range from about 2% to about 35%. In one embodiment, the degree of hydrolysis of the protein hydrolysate composition may range from about 0.2% to about 15%. In another embodiment, the degree of hydrolysis of the protein hydrolysate composition may range from about 0.2% to about 3%.

In general, the protein hydrolysate compositions, compared with the protein starting material, will comprise a mixture of polypeptide fragments of varying lengths and molecular weights. The molecular weight of the peptide fragments may range from 75 Daltons (i.e., free glycine) to greater than 100,000 Daltons, as measured by size exclusion chromatography. In general, the polypeptide fragments of the protein hydrolysate composition will have an average size of less than about 100,000 Daltons. In one embodiment, the average size of the polypeptide fragments of the protein hydrolysate composition may be from about 50,000 to about 100,000 Daltons. In another embodiment, the average size of the polypeptide fragments of the protein hydrolysate composition may be less than about 50,000 Daltons. In a further embodiment, the average size of the polypeptide fragments of the protein hydrolysate composition may be less than about 10,000 Daltons. In still another embodiment, the average size of the polypeptide fragments of the protein hydrolysate composition may be less than about 4,000 Daltons. In yet another embodiment, the average size of the polypeptide fragments of the protein hydrolysate composition may be less than about 2,000 Daltons.

### (III) Food Products Comprising A Protein Hydrolysate

A further aspect of the present invention is a food product comprising an edible material and a protein hydrolysate composition described herein.

The selection of a particular protein hydrolysate composition to combine with an edible material can and will vary depending upon the desired food product. In some embodiments, the protein hydrolysate composition may be derived from soy protein. In other embodiments, the protein hydrolysate composition may be derived from barley, canola, lupin, maize, oat, pea, potato, rice, wheat, animal, egg, or combinations thereof. In alternate embodiments, the protein hydrolysate composition may comprise a combination of different protein hydrolysates. For example, a soy protein hydrolysate composition may be combined with a maize protein hydrolysate combination. Alternatively, a soy protein hydrolysate composition may be combined with a canola protein hydrolysate composition and a wheat protein hydrolysate composition.

In still other embodiments, the protein hydrolysate composition may be derived from a combination of soy and at least one other protein source selected from the group consisting of barley, canola, lupin, maize, oat, pea, potato, rice, wheat, animal, dairy, and egg.

In further embodiments, the protein hydrolysate composition may further comprise at least one nonhydrolyzed protein selected from the group consisting of barley, canola, lupin, maize, oat, pea, potato, rice, wheat, animal, dairy, and egg. Non-limiting examples of suitable nonhydrolyzed proteins include dry milk powder, non-fat dry milk powder, milk proteins, acid casein, caseinate (e.g., sodium caseinate, calcium caseinate, etc.), whey protein concentrate, whey protein isolate, and soy protein isolate.

In some embodiments, the protein hydrolysate composition included in the food product may comprise "pre-peptides" that are converted into "active" peptides via proteolytic digestion in the stomach and/or intestine of the subject. In other embodiments, the protein hydrolysate composition comprises "active" peptides that require no additional proteolytic digestion in the stomach or intestines of the subject.

The selection of the appropriate edible material also will vary depending on the desired food product. The edible material may be a plant-derived material (e.g., a vegetable juice, a cereal product, etc,), an animal-derived material (e.g., a dairy product, an egg product, etc.), or a biomaterial (i.e., a protein, a carbohydrate, a lipid, etc.) isolated from a plant-derived material or an animal-derived material, and so forth.

In an embodiment, the food product may be a liquid beverage. Non-limiting examples of liquid beverages include fruit juices, fruit drinks, fruit-flavored drinks, vegetable drinks, nutritional drinks, energy drinks, sports drinks, soy milk drinks, flavored soy drinks, rice milk-based drinks, flavored milk drinks, yogurt-based drinks, infant formula, tea-based beverages, coffee-based beverages, meal replacement drinks, protein shakes, nutritional supplement beverages, weight management beverages, and combinations thereof.

The edible material comprising the beverage food product can and will vary. Non-limiting examples of suitable edible materials include fruit juices, vegetable juices, skim milk, reduced fat milk, 2% milk, whole milk, cream, evaporated milk, yogurt, buttermilk, chocolate, cocoa powder, coffee, tea, and so forth.

The beverage food product may further comprise sweetening agents (such as glucose, sucrose, fructose, maltodextrin, sucralose, corn syrup, honey, maple syrup, etc.), flavoring agents (e.g., chocolate, cocoa, chocolate flavor, vanilla extract, vanilla flavor, fruit flavors, etc), emulsifying or thickening agents (e.g., lecithin, carrageenan, cellulose gum, cellulose gel, starch, gum arabic, xanthan gum, and the like); stabilizing agents, lipid materials (e.g., canola oil, sunflower oil, high oleic sunflower oil, fat powder, etc.), preservatives (e.g., potassium sorbate, sorbic acid, and so forth), antioxidants (e.g., ascorbic acid, sodium ascorbate, etc.), coloring agents, vitamins, minerals, and combinations thereof.

In another embodiment, the food product may be a food bar, such as a granola bar, a cereal bar, a nutrition bar, or an energy bar. In still another embodiment, the food product may be a cereal-based product. Non-limiting examples of cereal-based food products include breakfast cereals, pasta, breads, baked products (i.e., cakes, pies, rolls, cookies, crackers), and snack products (e.g., chips, pretzels, etc.). The edible material of a cereal-based food product may be derived from wheat (e.g., bleached flour, whole wheat flour, wheat germ, wheat bran, etc.), corn (e.g., corn flour, cornmeal, cornstarch, etc.), oats (e.g., puffed oats, oatmeal, oat flour, etc), rice (e.g., puffed rice, rice flour, rice starch), and so forth. In yet another embodiment, the food product may be a "solid" dairy-based product. Non-limiting examples of suitable "solid" dairy-based food products include hard cheese products, soft cheese products, ice cream products, yogurt products, frozen yogurt products, whipped dairy-like products, sherbets, and the like. In an alternate embodiment, the food product may be a nutritional supplement. The nutritional supplement may be liquid or solid. In another alternate embodiment, the food product may be a meat product or a meat analog product. Examples of meat food products include, but are not limited to, processed meats, comminuted meats, and whole muscle meat products. The meat material may be animal meat or seafood meat. The meat analog may be a textured vegetable or dairy protein that mimics animal or seafood meat in texture. The meat analog may be part or all of the meat material in a meat food product.

## DEFINITIONS

To facilitate understanding of the invention, several terms are defined below.
The term "degree of hydrolysis" (DH) refers to the percent of specific peptide bonds that were hydrolyzed (that is, the number of cleaved out of the total number of peptide bonds present in the intact protein). The % DH was estimated using either the trinitrobenzene sulfonic acid (TNBS), or the ortho-phthaldialdehyde (OPA) method. These procedures are accurate, reproducible and generally applicable procedures for determining the degree of hydrolysis of food protein hydrolysates.

The term "endopeptidase" refers to an enzyme that hydrolyzes internal peptide bonds in oligopeptide or polypeptide chains. The group of endopeptidases comprises enzyme subclasses EC 3.4.21-25 (International Union of Biochemistry and Molecular Biology enzyme classification system).

The term "exopeptidase" refers to an enzyme that hydrolyzes proteins and/or peptides at or near their amino- or carboxyl termini. The group of exopeptidases comprises enzyme subclasses EC 3.4.11-18 (International Union of Biochemistry and Molecular Biology enzyme classification system).

A "food grade enzyme" is an enzyme that is generally recognized as safe (GRAS) approved and is safe when consumed by an organism, such as a human. Typically, the enzyme and the product from which the enzyme may be derived are produced in accordance with applicable legal and regulatory guidelines.

A "hydrolysate" is a reaction product obtained when a compound is cleaved through the effect of water. Protein hydrolysates occur subsequent to thermal, chemical, or enzymatic degradation. During the reaction, proteins are broken down into polypeptides, and/or free amino acids. These products may be soluble or insoluble in water or water-based buffer solutions.

The "OPA method" as used herein refers to the following procedure: 0.25gm of soy protein hydrolysate was dissolved in 50 ml of extraction buffer (1% SDS in 0.025 N Sodium Hydroxide, 0.6 mM DTT) by shaking for 5 minutes at 65°C, then cooled to 25°C. The sample was then centrifuged at 5000 x g for 5 minutes to remove any undissolved material. Next, 0.2 ml aliquots of the sample, serine standard (3.6 mM in deionized water), and extraction buffer (used as a blank) were transferred (in triplicate) to test tubes, diluted with 10 ml OPA color reagent (0.012M OPA, 0.1M sodium tetraborate, 2% SDS), and vortexed to mix. Reactions were allowed to proceed for 30 minutes, at which time absorbances were measured at 340 nm in a spectrophotometer. Means of each triplicate sample were used to determine %DH as described by Nielsen (Nielsen, P.M et al (2001) "Improved Method for Determining Food Protein Degree of Hydrolysis", J. Food Sci. 66(5):642-646).

A "peptide" is a short polymer of amino acids, generally 20 amino acids or less. A "polypeptide" is a polymer of amino acids greater than 20. Both of these polymers contain only primary structure. Polypeptides are formed initially during protein synthesis, and upon "folding" into their native state (i.e., the formation of secondary, tertiary, and quaternary structures) become proteins. In this application, reference to a polypeptide means the generation of a long chain polymer from the hydrolysis of a protein.

A "protein" is a polymer of amino acids that form an active molecule in its native (i.e., undenatured) state. The native state of a protein can have primary, secondary, tertiary, and or quaternary structures. The primary structure of a protein is its amino acid sequence. Proteins typically have secondary structure, which is formed from the interaction of intrachain amino acids. These structures are formed via hydrogen bonding, and are either alpha helixes or "sheets" of interacting amino acids known as beta sheets. Proteins also typically have tertiary structures as well. Tertiary structures are formed through the intrachain interaction of amino acid residues, and occur through ionic, hydrophobic, or other chemical interactions. Some proteins contain one or more "subunits", which interact molecularly to form quaternary structures. Protein subunits are composed of a single polypeptide chain and contain secondary and (usually) tertiary structures.

The terms "soy protein isolate" or "isolated soy protein," as used herein, refer to a soy material having a protein content of at least about 90% soy protein on a moisture free basis. A soy protein isolate is formed from soybeans by removing the hull and germ of the soybean from the cotyledon, flaking or grinding the cotyledon and removing oil from the flaked or ground cotyledon, separating the soy protein and carbohydrates of the cotyledon from the cotyledon fiber, and subsequently separating the soy protein from the carbohydrates.

The term "soy protein concentrate" as used herein is a soy material having a protein content of from about 65% to less than about 90% soy protein on a moisture-free basis. Soy protein concentrate may also contain soy cotyledon fiber, typically from about 3.5% up to about 20% soy cotyledon fiber by weight on a moisture-free basis. A soy protein concentrate is formed from soybeans by removing the hull and germ of the soybean, flaking or grinding the cotyledon and removing oil from the flaked or ground cotyledon, and separating the soy protein and soy cotyledon fiber from the soluble carbohydrates of the cotyledon.

The term "soy flour" as used herein, refers to full fat soy flour, enzyme-active soy flour, defatted soy flour, partially defatted soy flour, and mixtures thereof. Defatted soy flour refers to a comminuted form of defatted soybean material, preferably containing less than about 1% oil, formed of particles having a size such that the particles can pass through a No. 100 mesh (U.S. Standard) screen. The soy cake, chips, flakes, meal, or mixture of the materials are comminuted into soy flour using conventional soy grinding processes. Soy flour has a soy protein content of about 49% to about 65% on a moisture free basis. Preferably the flour is very finely ground, most preferably so that less than about 1% of the flour is retained on a 300 mesh (U.S. Standard) screen. Full fat soy flour refers to ground whole soybeans containing all of the original oil, usually 18% to 20%. The flour may be enzyme-active or it may be heat-processed or toasted to minimize enzyme activity. Enzyme-activity soy flour refers to a full fat soy flour that has been minimally heat-treated in order not to neutralize its natural enzymes.

The term "soymilk" as used herein, refers to an aqueous mixture of any one or more of the following, finely ground soybeans, soy flour, soy flakes, soy concentrate, isolated soy protein, soy whey protein, and aqueous extracts of any one or more of the following: soybeans, soy flakes and soy flour where insoluble material has been removed. Soymilk may comprise additional components including but not limited to fats, carbohydrates, sweeteners, colorants, stabilizers, thickeners, flavorings, acids, and bases.

The term "soymilk powder" as used herein, refers to a dewatered soymilk. Soymilk may be dewatered by many processes that include but are not limited to spray drying, tray drying, tunnel drying, and freeze drying.

The term " simplified trinitrobenzene sulfonic acid (S-TNBS) method" as used herein, refers to an accurate, reproducible and generally applicable procedure for determining the degree of hydrolysis of food protein hydrolysates. For this, 0.1 g of the soy protein hydrolysate was dissolved in 100 mL of 0.025 N NaOH. An aliquot (2.0 mL) of the hydrolysate solution was mixed with 8 mL of 0.05 M sodium borate buffer (pH 9.5). Two mL of the buffered hydrolysate solution was treated with 0.20 mL of 10% trinitrobenzene sulfonic acid, followed by incubation in the dark for 15 minutes at room temperature. The reaction was quenched by adding 4 mL of a 0.1 M sodium sulfite-0.1 M sodium phosphate solution (1:99 ratio), and the absorbance was read at 420 nm. A 0.1 mM glycine solution was used as the standard. The following calculation was used to determine the percent recovery for the glycine standard solution: [(absorbance of glycine at 420 nm - absorbance of blank at 420 nm) x (100/0.710)]. Values of 94% or higher were considered acceptable. (Jens Adler-Nissen (1979) "Determination of the Degree of Hydrolysis of Food Protein Hydrolysates by Trinitrobenzenesulfonic Acid," J. Agric. Food Chem., 27(6):1256-1262).

When introducing elements of the present invention or the preferred embodiment(s) thereof, the articles "a," "an," "the," and "said" are intended to mean that there are one or more of the elements. The terms "comprising," "including," and "having" are intended to be inclusive and mean that there may be additional elements other than the listed elements.

As various changes could be made in the above compounds, products and methods without departing from the scope of the invention, it is intended that all matter contained in the above description and in the examples given below, shall be interpreted as illustrative and not in a limiting sense.

## EXAMPLES

The following examples illustrate various iterations of the invention.

### Example 1. Initial Screen for CCK Releasing Activity.

A cell-based assay was used to determine whether complex mixtures of soy proteins/soy peptides stimulated the release of CCK. Initially 40 different samples were assayed to determine which had the highest CCK stimulating activity. Cell viability was also assessed after exposure to the various preparations.

An aliquot (0.1 g) of each freeze-dried sample was reconstituted in 5 ml of phosphate buffered saline (PBS; 137 mM NaCl, 2.7 mM KCl, 4.3 mM $Na_2HPO_4$, 1.4 mM $KH_2PO_4$, pH 7.2) to a stock concentration of 20 mg/ml (w/v). After gentle mixing for about 15 minutes, the samples were allowed to hydrate overnight at 4°C, and then centrifuged at 16,000 x g for 30 minutes at 4°C to remove insoluble material. The supernatant fractions were diluted 1:10 in serum-free culture medium and assayed in triplicate for CCK release at a final concentration of about 2 mg/ml. STC-1 cells, a mouse enteroendocrine cell line that displays many features of native intestinal CCK-producing cells (Rindi et al. 1990, Am. J. Pathol. 136:1349-1364; Chang et al., 1994, Biochim. Biophys. Acta 1221:339-437), were exposed to the soy protein samples for 4 hour under standard conditions. The negative control was bovine serum albumin (BSA) at 2 mg/ml (w/v) in serum-free culture medium and the positive control was 100 nM PMA in 2 mg/ml BSA (w/v) in serum-free culture medium. The cell-conditioned medium was removed and CCK levels were measured using a competitive enzyme assay. Cell viability was assessed using the (3-[4,5-dimethylthiazol-2-yl]-2,5-diphenyltetrazolium bromide (MTT) assay (Vellomen K-S, Honkakoski P and Urtti A (2004) Substrates and Inhibitors of Efflux Proteins Interfere with the MTT Assay in Cells and May Lead to Underestimation of Drug Toxicity, Eur. J. Pharm. Sci. 23:181-188).

All of the initial samples with CCK stimulating activity were hydrolysates. In the initial sample set, the non-hydrolyzed protein samples did not stimulate CCK release over basal levels. This can be seen for intact soy and caseinate protein in **Figure 1.** No significant loss in cell viability or metabolic activity was detected upon exposure to any of the initial samples.

The 40 samples were re-screened with this cell-based assay using fresh preparations of the samples. While the absolute amount of CCK released varied, the same 11 samples were ranked as the top stimulators (% of control PMA was 30% or greater).

**Example 2. Fractionation of a Potent CCK Releasing Sample.**

Sample 9 (i.e., SUPRO®950/FXP950, which is isolated soy protein hydrolyzed with ALCALASE®) had one of the highest CCK-releasing activities. To estimate the molecular weights of the peptides in this sample, it was fractionated by tangential flow filtration. For this, a 5% slurry of the sample was fractionated using a tangential flow filtration unit equipped with a 100 kDa MWCO flat sheet membrane (Lab 20, Alfa Laval, UK). The retentate was collected (i.e., a greater than 100 kDa fraction) and the permeate was fractioned using the same filtration unit equipped with a 10 kDa MWCO flat sheet membrane to form a 10-100 kDa fraction and a less than 10 kDa fraction. The fractions were lyophilized, resuspended in PBS, diluted in serum-free culture medium, and four concentrations (w/v) of each were assayed for CCK-releasing activity in STC-1 cells as detailed above.

Table 1 presents the results. The 10-100 kDa and less than 10 kDa fractions had the highest CCK release stimulating activity. The fractions were also resolved by SDS-PAGE (see **Figure 2**) and low molecular weight peptides were present in every fraction. This finding suggests that low and high molecular weight molecules may be interacting through hydrophobic or other interactions and, consequently, are not well separated by this method. It is clear, however, that low molecular weight peptides induce CCK release and they account for the activity observed in the higher molecular weight fractions.

**Table 1.** CCK Releasing Activity of Fractionated Sample.

| # | | | CCK Release (ng/ml) mean $\pm$ sem | CCK Release (% PMA release) |
|---|---|---|---|---|
| 41 | Starting sample (SUPRO®950/FXP950*) | | | |
| | | 8 mg/ml | 0.166 $\pm$ 0.017 | 95.7% |
| | | 2 mg/ml | 0.117 $\pm$ 0.012 | 53.8% |
| | | 0.5 mg/ml | 0.069 $\pm$ 0.005 | 12.8% |
| | | 0.125 mg/ml | 0.056 $\pm$ 0.003 | 1.7% |
| 42 | >100 kDa fraction | | | |
| | | 8 mg/ml | 0.161 $\pm$ 0.006 | 91.5% |
| | | 2 mg/ml | 0.105 $\pm$ 0.004 | 43.6% |
| | | 0.5 mg/ml | 0.073 $\pm$ 0.002 | 16.2% |
| | | 0.125 mg/ml | 0.059 $\pm$ 0.002 | 4.3% |
| 43 | 10-100 kDa fraction | | | |
| | | 8 mg/ml | 0.204 $\pm$ 0.003 | 128.2% |
| | | 2 mg/ml | 0.141 $\pm$ 0.003 | 74.4% |
| | | 0.5 mg/ml | 0.079 $\pm$ 0.023 | 21.4% |
| | | 0.125 mg/ml | 0.085 $\pm$ 0.021 | 26.5% |
| 44 | < 10 kDa fraction | | | |
| | | 8 mg/ml | 0.183 $\pm$ 0.015 | 110.3% |
| | | 2 mg/ml | 0.122 $\pm$ 0.016 | 58.1% |
| | | 0.5 mg/ml | 0.100 $\pm$ 0.007 | 39.3% |
| | | 0.125 mg/ml | 0.079 $\pm$ 0.005 | 21.4% |
| | Negative control (BSA) | | 0.054 $\pm$ 0.002 | |
| | Positive control (PMA) | | 0.171 $\pm$ 0.003 | |
| * ISP treated with ALCALASE® | | | | |

**Example 3. CCK Releasing Activity of a Potent CCK Releasing Hydrolysate Fraction After Pepsin and Pepsin-Pancreatin Digestion to Mimic In Vivo Digestion in the Intestine**

**Figure 3** shows the stimulation of CCK release by pepsin and pepsin-pancreatin digested preparations of the 10 - 100 kDa fraction of SUPRO®950/FXP950, a hydrolyzed protein preparation described in Example 2, showing that the CCK releasing activity of this peptide hydrolysate fraction was maintained after digestion of this fraction by enzymes known to be present in the digestive track of humans and other animals. The digestion method with pepsin and pepsin-pancreatin, to mimic in vivo stomach and upper intestinal digestion, is a modification of the previously published procedures of Schasteen (Shasteen, C.S., et al., (2007) Correlation of an Immobilized Digestive Enzyme Assay With Poultry True Amino Acid Digestibility for Soybean Meal. Poultry Science 86(2), 343-348) and Higaki (Higaki, N., et al, (2006) Biosci. Biotechnol. Biochem. 70(12), 2844-2852). Protein samples were solubilized in 20 volumes of 0.01 M HCl and digested by pepsin (Sigma-Aldrich #P7012) at an enzyme-substrate ratio of 1:200 (w/w), pH 2.3 and 37°C for 4 hour. After the pepsin digestion, 2.5 M NaOH was added to the mixture to adjust the pH to 8.0, and pancreatin (Sigma-Aldrich #P3292) was added at a ratio of 1:200 (w/w) and digestion was continued for another 4 to18 hours. Degree of hydrolysis was determined by the reaction of primary amine groups with o-phthalaldehyde (OPA) vs. total amount of primary amine present in sample after acid hydrolysis (110°C for 24 hours). The protein hydrolysates were added to the media of STC-1 cells at a protein concentration from 0.5 to 8 mg/mL, and the enzyme controls were added at equivalent dilutions of the control reaction mixture (which included the enzyme(s) in the absence of protein substrate). Plotted is the absolute concentration of CCK (ng/mL) released into the media of STC-1 cells stimulated by the pepsin and pepsin-pancreatin treated protein hydrolysate samples. The concentration of CCK stimulated by the positive control 100 nM PMA and negative control 2 mg/mL BSA are shown by the arrows marked 'PMA Control' and 'Baseline CCK Release', respectively, in **Figure 3.**

**Example 4. CCK-Releasing Activity of Soy Protein Hydrolysates.**

The CCK releasing activity of several different soy protein hydrolysates with various degrees of hydrolysis was also analyzed. For this, isolated soy protein was hydrolyzed with ALCALASE®, bromelain, serine protease, Alcalase® 2, S2, MP1, TL1, and ASP-1. The amount of enzyme added and/or the duration of the incubation period were adjusted to give various degrees of hydrolysis. STC-1 cells were exposed to the different hydrolysates diluted to a final protein concentration of about 2 mg/mL. CCK release was assayed as described above. **Figure 4** illustrates the stimulation of CCK release for different soy protein hydrolysate preparations. Plotted is the % CCK released into the media of STC-1 stimulated by the different soy protein hydrolysates generated with the different enzymes as indicated in **Figures 4A-4H** and with different % degrees of hydrolysis (%DH) obtained with the various incubation conditions compared to the % CCK released by PMA, which was set at 100%. %CCK released into the cell culture media is calculated as follows:

$$\% \text{ CCK release} = \frac{(\text{ng CCK}_{\text{sample well}} - \text{ng CCK}_{\text{BSA control well}})}{(\text{ng CCK}_{\text{PMA well}} - \text{ng CCK}_{\text{BSA control well}})} \times 100$$

PMA induces CCK through a direct stimulation of protein kinase C (PKC). Each soy protein hydrolysate was added to STC-1 cells at a final protein concentration of approximately 2 mg/mL. The relative ability of the soy hydrolysates to induce CCK release by the STC-1 cells is dependent on the enzyme and degree of hydrolysis.

**Experimental Example 1. Food Bar Containing Soy Protein Hydrolysates.**

In this Experimental Example, samples of food bars comprising proteinaceous material and sugar syrups are produced using ingredients listed in Table 2 below.

To obtain the food bars, a first mixture is produced in a Hobart mixer (N50 5-Quart Mixer, Legacy® Countertop Mixer, Legacy® Floor Mixer, Hobart Corporation, Tory, OH). Mix sugar type syrup, crystalline sugar, glycerin, liquid oil, liquid inclusions, gums, and natural or artificial flavors in the bowl. Mix the slurry mixture for 1 minute at a speed setting of 2. Scrape the bowl with a spatula so the side of the bowl is clean.

After mixing the slurry for 1 minute, add soy protein isolate, soy protein hydrolysate, and particle inclusions to the mixing bowl. Mix mixture for 1 minute at a speed setting of 1. Scrape the bowl with spatula until side of the bowl is clean. Mix for another 30 seconds at a speed setting of 1. The resulting dough is then sheeted out onto a slab and bars are cut into pieces weighing from about 20 grams to about 70 grams.

Table 2 Basic Food Bar Formulation

| Ingredients | Range (in grams) |
| --- | --- |
| Sugar type syrup | 10-40% |
| Sugar, crystalline | 2-10% |
| Glycerin | 1-10% |

(continued)

| Ingredients | Range (in grams) |
|---|---|
| Liquid Oil | 1-10% |
| Liquid inclusions | |
| Gums | 0.1-5% |
| Natural or Artificial Flavor | 0.01-3% |
| Soy Protein Isolate | 1-40% |
| Soy Protein Hydrolysate | 1-40% |
| Particle Inclusions | 1-10% |
| | |
| Total | |

**Experimental Example 2: Acid Beverage Containing Soy Protein Hydrolysates.**

An acid beverage according to the present invention is prepared. 130.0 parts soy protein ingredient and 8539 parts deionized water are added to a vessel. The contents are mixed under high shear until evenly dispersed. The dispersion is then heated to 74°C to 79°C (165°F to 175°F) and mixed for an additional 10 minutes. Then added with mixing are 1180 parts high fructose corn syrup, 131 parts apple juice concentrate (68 Brix) and 20.0 parts anhydrous citric acid. The pH is adjusted to 3.8-4.0 with 85% citric acid. The contents are homogenized at 2500 pounds per square inch in the first stage and at 500 pounds per square inch in the second stage followed by pasteurization at 107°C for 7 seconds. Bottles are hot filled with the beverage and then placed in an ice bath to bring the temperature of the beverage to about room temperature and placed in the refrigerator.

**Experimental Example 3. A Dry Blend Containing the Soy Protein Hydrolysates.**

The following Experimental Example illustrates the preparation of the dry blend containing the protein of this invention with components listed in Table 3 below.

Table 3

| Component | Parts by Weight | Grams per Serving |
|---|---|---|
| Soy Protein Hydrolysates | 56.85 | 16.89 |
| Fructose | 20.23 | 6.01 |
| Sucrose | 20.22 | 6.01 |
| Dry Cream Extract | 1.01 | 0.30 |
| Ice Cream Vanilla Flavor | 1.35 | 0.40 |
| Sodium Chloride | <u>0.34</u> | <u>0.10</u> |
| Total | 100.00 | 29.71 |

Ingredients are added to a vessel and mixed to form a dry blend.

**Experimental Example 4 - A beverage containing the dry blend as prepared in Experimental Example 3**

A ready to drink beverage is prepared by adding a dry blend as prepared in Experimental Example 3 to a liquid. Order of addition is of no importance.
Within the ready to drink beverage, the liquid is present at from about 85% up to about 95% by weight of the total composition, and the pH of the ready to drink beverage is from about 6.8 up to about 7.4.
Experimental Example 4 is the inventive ready to drink beverage prepared by adding 29.71 grams of the product of Experimental Example 3 to 240 ml of skim milk. The contents are blended for 30 seconds.

**Experimental Example 5 - A beverage containing the dry blend as prepared in Experimental Example 3**

A ready to drink beverage is prepared by adding a dry blend as prepared in Experimental Example 3 to a liquid. Order of addition is of no importance.

Within the ready to drink beverage, the liquid is present at from about 85% up to about 95% by weight of the total composition, and the pH of the ready to drink beverage is from about 6.8 up to about 7.4.

Experimental Example 5 is the inventive ready to drink beverage prepared by adding 29.71 grams of the product of Experimental Example 3 to 240 ml of water. The contents are blended for 30 seconds.

**Experimental Example 6 Unflavored Low Fat Soymilk Containing the Soy Protein Hydrolysates**

Serving size: 8.5g protein/250g.

Table 4 Formula for Lowfat Soymilk

| Ingredients | % in formula |
|---|---|
| Distilled Water | 89.4 |
| Potassium citrate | 0.25 |
| Soy protein hydrolysate* | 3.8-4.2 |
| Maltodextrin | 4-4.4 |
| Sugar | 1.4 |
| High Oleic Sunflower oil | 0.72 |
| Carrageenan | 0.03 |
| * Percentage of soy protein hydrolysate used in formula was adjusted depending on protein content as is | |

Disperse citrate in 60°C (140°F) water. Increase mixing speed and disperse protein into water. After protein is thoroughly dispersed increase slurry temperature to 75°C (167°F), reduce mixing speed and continue mixing 10 minutes. Preblend maltodextrin, sugar and carrageenan, add to protein slurry and continue mixing at low speed for 5 minutes. Add sunflower oil to slurry and continue mixing at slow speed until homogenous for approximately 3 minutes. Adjust slurry pH using 45% potassium hydroxide to between 7.0 and 7.2. Process as follows: homogenization, pasteurization and cooling. Heat product to 72°C (162°F) and homogenize at 750 psi, second stage; 2250 psi, first stage. Pre-heat slurry to 100°C (212°F) and UHT at 141°C (286°F) for 6 seconds. Cool product to 31°C (88°F) and package in sterilized bottles. Store refrigerated.

**Experimental Example 7 Unflavored Beverage Containing 50% Soy Protein Hydrolysates and 50% Skim Milk.**

Serving size: 8g protein/260g

Table 5 Unflavored Beverage formula

| Ingredients | % in formula |
|---|---|
| Distilled Water | 43.9 |
| Soy protein hydrolysates* | 1.71-1.9 |
| Skim milk | 50 |
| Maltodextrin | 1.95-2.12 |
| Sugar | 1.0 |
| High Oleic Sunflower oil | 0.84 |
| Sodium citrate, dehydrate | 0.05 |
| Magnesium phosphate, dibasic | 0.038 |

(continued)

| Ingredients | % in formula |
|---|---|
| Cellulose gel | 0.25 |
| Carrageenan | 0.02 |
| Vitamin/mineral premix | 0.006 |
| * Percentage of soy protein hydrolysates used in formula was adjusted depending on protein content as is | |

Disperse citrate in all deionized water at 15°C (59°F) using moderate speed mixing. Disperse SUPRO® Plus in water. After all lumps are dispersed, heat slurry to 77°C (170°F) and continue mixing at slow speed for 10 minutes. Dry blend maltodextrin, sugar, vit/min premix, magnesium phosphate, cellulose and carrageenan. Add dry blend to protein slurry and continue mixing 5 minutes. Add sunflower oil and continue mixing 3 minutes. Measure slurry pH and adjust pH if necessary to pH 6.9 to 7.1 using either 50% citric acid solution or 1 N NaOH. Heat skim milk slowly to 72°C (162°F) and add protein slurry to heated skim milk. Add flavoring agents and mix until completely incorporated into slurry. Blend for 3 minutes with slow mixing and record final slurry pH. Process as follows: Heat product to 72°C (162°F) and homogenize at 750 psi, second stage, 2250 psi, first stage. Pre-heat slurry to 104°C (220°F) and UHT at 141 °C (286°F) for 6 seconds. Cool product to 31°C (88°F) and package. Store refrigerated.

**Experimental Example 7 Vanilla Flavored Weight Management Beverage Containing Calcium Caseinate and Non-Fat Dry Milk (NFDM) and Soy Protein Hydrolysate as the Protein Sources**

Serving size: 10g protein/11 oz

Table 6 Vanilla Flavored Weight Management Beverage Formula

| Ingredients | % in formula | |
|---|---|---|
| | All milk protein | Soy containing 20% Ca caseinate replacement |
| Distilled Water | 82.4 | 82.5 |
| Soy protein hydrolysate | 0.0000 | 0.68-0.71 |
| NFDM | 6.39 | 6.39 |
| Sucrose | 7.0 | 7.0 |
| Gum Arabic | 1.31 | 1.31 |
| Calcium Caseinate (85.5%) | 0.68 | 0 |
| Cellulose Gel | 0.35 | 0.35 |
| Canola Oil | 0.77 | 0.74 |
| Potassium Citrate | 0.14 | 0.13 |
| Sodium Citrate | 0.05 | 0.04 |
| Lecithin | 0.07 | 0.07 |
| Carrageenan | 0.04 | 0.04 |
| Calcium Carbonate | 0.06 | 0.03 |
| Magesium Carbonate | 0 | 0.02 |
| Magnesium Phosphate, dibasic | 0.25 | 0.21 |
| Vit/Min. premix | 0.07 | 0.07 |
| Vanilla flavor | 0.40 | 0.40 |

Prepare a pre-blend as follows: mix the carrageenan and cellulose with a small portion of the formula sucrose.

Mix the gum arabic and remaining sucrose. Mix canola oil.

Then blend: Add the potassium and sodium citrate to water using high shear mixing. Then disperse the preblended carrageenan and cellulose. Mix for 5 minutes. Disperse the soy protein, calcium caseinate and NFDM and begin heating to 65°C (150°F). Hydrate for 15 minutes, reduce mixing speed after reaching 65°C (150°F). Heat canola oil/emulsifier blend to about 70°C (158°F) to dissolve emulsifiers, this may require some mixing. Then add hot oil mix to batch tank, blend 5 minutes, foam will disperse. Add sucrose/gum arabic blend, calcium carbonate, magnesium phosphate, magnesium carbonate and vitamin premix, mix for 10 minutes. Add vanilla flavor and adjust pH with 45% KOH to 7.0 - 7.2. UHT 286F/6seconds, 2500/500 psi

**Experimental Example 8 Dry Blended Beverage Performance Beverage Containing Soy Protein Hydrolysates.**

Table 7 Dry-Blended Performance Beverage Formula

| Ingredients | % in formula |
|---|---|
| Soy protein hydrolysates | 16.2-16.9 |
| Whey Protein Isolate (92.7% protein as is) | 15.81 |
| Sugar | 3.25 |
| Fructose | 3.25 |
| Cocoa | 3.00 |
| Fat Powder | 1.40 |
| Xanthan Gum | 0.40 |
| Vitamin Premix, | 0.06 |
| Sucralose | 0.04 |
| Sweetness Enhancer, | 0.30 |
| Chocolate flavor | 0.65 |
| Cream | 0.20 |
| Total | 44.8 - 45.3 |

Clean and sanitize mixer. Sieve soy protein and cocoa powder. Mix all ingredients for 15 minutes at medium speed. Store dry powder in sanitized containers.

**Claims**

1. A soluble fraction of a protein hydrolysate composition, the composition comprising a mixture of polypeptide fragments that have an average size of less than about 100,000 Daltons, said mixture comprising a 10,000-100,000 Daltons polypeptide fraction, wherein a concentration of at least about 0.5 mg/mL of the protein hydrolysate composition stimulates cholecystokinin releasing activity with a potency substantially similar to greater than 50% of cholecystokinin released by STC-1 cells stimulated with 100 nM of phorbol 12-myristate-13-acetate for 4 hours.

2. The protein hydrolysate composition of claim 1, wherein the protein hydrolysate composition has a degree of hydrolysis from about 0.05% to about 35%.

3. The protein hydrolysate composition of claim 1, wherein the protein hydrolysate composition is derived from a protein material selected from the group consisting of soy, barley, canola, lupin, maize, oat, pea, potato, rice, wheat, egg, animal, and combinations thereof.

4. The protein hydrolysate composition of claim 1, wherein the protein hydrolysate composition is derived from soy protein material in combination with a protein material selected from the group consisting of barley, canola, lupin, maize, oat, pea, potato, rice, wheat, egg, dairy, animal, and combinations thereof.

**5.** The protein hydrolysate composition of claim 1, wherein the protein hydrolysate composition is derived from soy protein material, and the protein hydrolysate composition has a degree of hydrolysis of from about 0.05% to about 35%.

**6.** A method for increasing the cholecystokinin releasing activity of a cell, the method comprising contacting the cell with a soluble fraction of a protein hydrolysate composition, the composition comprising a mixture of polypeptide fragments that have an average size of less than about 100,000 Daltons, said mixture comprising a 10,000-100,000 Daltons polypeptide fraction, wherein a concentration of at least about 0.5 mg/mL of the protein hydrolysate composition stimulates cholecystokinin releasing activity with a potency substantially similar to greater than 50% of cholecystokinin released by STC-1 cells stimulated with 100 nM of phorbol 12-myristate-13-acetate for 4 hours.

**7.** The method of claim 6, wherein the protein hydrolysate composition has a degree of hydrolysis from about 0.05% to about 35%.

**8.** The method of claim 6, wherein the protein hydrolysate composition is derived from soy protein material in combination with a protein material selected from the group consisting of barley, canola, lupin, maize, oat, pea, potato, rice, wheat, egg, dairy, animal, and combinations thereof.

**9.** The method of claim 6, wherein the protein hydrolysate composition is derived from soy protein material.

**10.** The method of claim 9, wherein the soy protein material is selected from the group consisting of soy extract, soy milk, soy milk powder, soy curd, defatted soy flour, partially defatted soy flour, full fat soy flour, isolated soy protein, soy protein concentrate, and a combination thereof.

**11.** The method of claim 6, wherein the soy protein hydrolysate composition is produced by subjecting a protein starting material to enzymatic digestion.

**12.** The method of claim 11, wherein the enzyme is an endopeptidase selected from the group consisting of serine protease (SP1) from Nocardiopsis prasina, serine protease from Bacillus licheniformis, subtilisin protease from Bacillus licheniformis, trypsin-like protease from Fusarium oxysporum, lysyl endopeptidase from Achromobacter lyticus, subtilisin protease 2, metallo protease 1 , aspartate protease 1 , bromelain, subtilisin, and combinations thereof.

**13.** The method of claim 12, wherein the enzyme further comprises an exopeptidase.

**14.** A method for promoting satiety in a subject, the method comprising administering to the subject an amount of a soluble fraction of a protein hydrolysate composition, the composition comprising a mixture of polypeptide fragments that have an average size of less than about 100,000 Daltons, and a degree of hydrolysis from about 0.05% to about 35%, said mixture comprising a 10,000-100,000 Daltons polypeptide fraction, wherein a concentration of at least about 0.5 mg/mL of the protein hydrolysate composition stimulates cholecystokinin releasing activity with a potency substantially similar to greater than 50% of cholecystokinin released by STC-1 cells stimulated with 100 nM of phorbol 12-myristate-13-acetate for 4 hours, wherein the amount administered results in a feeling of satiety by the subject.

**15.** A food product, the food product comprising:

(a) an edible material; and
(b) a soluble fraction of a protein hydrolysate composition, the composition comprising a mixture of polypeptide fragments that have an average size of less than about 100,000 Daltons, said mixture comprising a 10,000-100,000 Daltons polypeptide fraction, wherein a concentration of at least about 0.5 mg/mL of the protein hydrolysate composition stimulates cholecystokinin releasing activity with a potency substantially similar to greater than 50% of cholecystokinin released by STC-1 cells stimulated with 100 nM of phorbol 12-myristate-13-acetate for 4 hours.

**Patentansprüche**

**1.** Lösliche Fraktion einer Proteinhydrolysatzusammensetzung, wobei die Zusammensetzung ein Gemisch von Polypeptidfragmenten umfasst, die eine durchschnittliche Größe von weniger als etwa 100000 Dalton aufweisen, wobei das Gemisch eine 10000-100000 Dalton Polypeptidfraktion umfasst, wobei eine Konzentration von wenigstens etwa 0,5 mg/ml der Proteinhydrolysatzusammensetzung eine Cholecystokinin-freisetzende Aktivität stimuliert, die eine Wirksamkeit aufweist, die im Wesentlichen gleich ist der Wirksamkeit von mehr als 50 % Cholecystokinin, freigesetzt aus mit 100 nM Phorbol-12-myristat-13-acetat 4 Stunden lang stimulierten STC-1 Zellen.

2. Proteinhydrolysatzusammensetzung nach Anspruch 1, wobei die Proteinhydrolysatzusammensetzung einen Hydrolysegrad von etwa 0,05 % bis 35 % aufweist.

3. Proteinhydrolysatzusammensetzung nach Anspruch 1, wobei die Proteinhydrolysatzusammensetzung abgeleitet ist aus einem Proteinmaterial ausgewählt aus der Gruppe bestehend aus Soja, Gerste, Canola, Lupine, Mais, Hafer, Erbse, Kartoffel, Reis, Weizen, Ei, Tier und Kombinationen davon.

4. Proteinhydrolysatzusammensetzung nach Anspruch 1, wobei die Proteinhydrolysatzusammensetzung abgeleitet ist aus Sojaproteinmaterial in Kombination mit einem Proteinmaterial ausgewählt aus der Gruppe bestehend aus Gerste, Canola, Lupine, Mais, Hafer, Erbse, Kartoffel, Reis, Weizen, Ei, Milchprodukten, Tier und Kombinationen davon.

5. Proteinhydrolysatzusammensetzung nach Anspruch 1, wobei die Proteinhydrolysatzusammensetzung von Sojaproteinmaterial abgeleitet ist und die Proteinhydrolysatzusammensetzung einen Hydrolysegrad von etwa 0,05 % bis etwa 35 % aufweist.

6. Verfahren zum Erhöhen der Cholecystokininfreisetzenden Aktivität einer Zelle, wobei das Verfahren das in Kontakt bringen der Zelle mit einer löslichen Fraktion einer Proteinhydrolysatzusammensetzung umfasst, wobei die Zusammensetzung ein Gemisch von Polypeptidfragmenten umfasst, die eine durchschnittliche Größe von weniger als etwa 100000 Dalton aufweisen, wobei das Gemisch eine 10000-100000 Dalton Polypeptidfraktion umfasst, wobei eine Konzentration von wenigstens etwa 0,5 mg/ml der Proteinhydrolysatzusammensetzung eine Cholecystokinin-freisetzende Aktivität stimuliert, die eine Wirksamkeit aufweist, die im Wesentlichen gleich ist der Wirksamkeit von mehr als 50 % Cholecystokinin, freigesetzt aus mit 100 nM Phorbol-12-myristat-13-acetat 4 Stunden lang stimulierten STC-1 Zellen.

7. Verfahren nach Anspruch 6, wobei die Proteinhydrolysatzusammensetzung einen Hydrolysegrad von etwa 0,05 % bis etwa 35 % aufweist.

8. Verfahren nach Anspruch 6, wobei die Proteinhydrolysatzusammensetzung abgeleitet ist aus Sojaproteinmaterial in Kombination mit einem Proteinmaterial ausgewählt aus der Gruppe bestehend aus Gerste, Canola, Lupine, Mais, Hafer, Erbse, Kartoffel, Reis, Weizen, Ei, Milchprodukten, Tier und Kombinationen davon.

9. Verfahren nach Anspruch 6, wobei die Proteinhydrolysatzusammensetzung aus Sojaproteinmaterial abgeleitet ist.

10. Verfahren nach Anspruch 9, wobei das Sojaproteinmaterial ausgewählt ist aus der Gruppe bestehend aus Sojaextrakt, Sojamilch, Sojamilchpulver, Sojaquark, fettarmem Sojamehl, teilentfettetem Sojamehl, Vollfett-Sojamehl, isoliertem Sojaprotein, Sojaproteinkonzentrat und einer Kombination davon.

11. Verfahren nach Anspruch 6, wobei die Sojaproteinhydrolysatzusammensetzung dadurch hergestellt wird, dass Proteinstartmaterial einem enzymatischen Verdau unterzogen wird.

12. Verfahren nach Anspruch 11, wobei das Enzym eine Endopeptidase ist, die ausgewählt ist aus der Gruppe bestehend aus Serinprotease (SP1) aus Nocardiopsis prasina, Serinprotease aus Bacillus licheniformis, Subtilisinprotease aus Bacillus licheniformis, Trypsin-ähnlicher Protease aus Fusarium oxysporum, Lysylendopeptidase aus Achromobacter lyticus, Subtilisinprotease 2, Metalloprotease 1, Aspartatprotease 1, Bromelain, Subtilisin und Kombinationen davon.

13. Verfahren nach Anspruch 12, wobei das Enzym ferner eine Exopeptidase umfasst.

14. Verfahren zum Fördern der Sättigung einer Zielperson, wobei das Verfahren die Verabreichung einer Menge einer löslichen Fraktion einer Proteinhydrolysatzusammensetzung an die Zielperson umfasst, wobei die Zusammensetzung ein Gemisch von Polypeptidfragmenten umfasst, die eine durchschnittliche Größe von weniger als etwa 100000 Dalton und einen Hydrolysegrad von etwa 0,05 % bis etwa 35 % aufweisen, wobei das Gemisch eine 10000-100000 Dalton Polypeptidfraktion umfasst, wobei eine Konzentration von wenigstens etwa 0,5 mg/ml der Proteinhydrolysatzusammensetzung eine Cholecystokinin-freisetzende Aktivität stimuliert, die eine Wirksamkeit aufweist, die im Wesentlichen gleich ist der Wirksamkeit von mehr als 50 % Cholecystokinin, freigesetzt aus mit 100 nM Phorbol-12-myristat-13-acetat 4 Stunden lang stimulierten STC-1 Zellen, wobei die verabreichte Menge bei der Zielperson zu einem Sättigungsgefühl führt.

15. Nahrungsmittelprodukt, wobei das Nahrungsmittelprodukt umfasst:

(a) ein essbares Material; und
(b) eine lösliche Fraktion einer Proteinhydrolysatzusammensetzung, wobei die Zusammensetzung ein Gemisch von Polypeptidfragmenten umfasst, die eine durchschnittliche Größe von weniger als etwa 100000 Dalton aufweisen, wobei das Gemisch eine 10000-100000 Dalton Polypeptidfraktion umfasst, wobei eine Konzentration von wenigstens etwa 0,5 mg/ml der Proteinhydrolysatzusammensetzung eine Cholecystokinin-freisetzende Aktivität stimuliert, die eine Wirksamkeit aufweist, die im Wesentlichen gleich ist der Wirksamkeit von mehr als 50 % Cholecystokinin, freigesetzt aus mit 100 nM Phorbol-12-myristat-13-acetat 4 Stunden lang stimulierten STC-1 Zellen.

## Revendications

1. Une fraction soluble d'une composition d'hydrolysat de protéine, la composition comprenant un mélange de fragments polypeptidiques ayant une taille moyenne inférieure à environ 100 000 Daltons, ledit mélange comprenant une fraction polypeptidique de 10 000 à 100 000 Daltons, dans laquelle une concentration d'au moins environ 0,5 mg/ml de la composition d'hydrolysat de protéine stimule l'activité de libération de cholécystokinine avec une activité sensiblement similaire à plus de 50 % de cholécystokinine libérés par des cellules STC-1 stimulées par 100 nM de 12-myristate-13-acétate de phorbol pendant 4 heures.

2. La composition d'hydrolysat de protéine selon la revendication 1, dans laquelle la composition d'hydrolysat de protéine a un degré d'hydrolyse d'environ 0,05 % à environ 35 %.

3. La composition d'hydrolysat de protéine selon la revendication 1, dans laquelle la composition d'hydrolysat de protéine est dérivée d'une matière protéique choisie dans le groupe constitué du soja, de l'orge, du canola, du lupin, du maïs, de l'avoine, du pois, de la pomme de terre, du riz, du blé, des oeufs, d'un animal et de leurs combinaisons.

4. La composition d'hydrolysat de protéine selon la revendication 1, dans laquelle la composition d'hydrolysat de protéine est dérivée d'une matière protéique à base de soja en combinaison avec une matière protéique choisie dans le groupe constitué de l'orge, du canola, du lupin, du maïs, de l'avoine, du pois, de la pomme de terre, du riz, du blé, des oeufs, d'un produit laitier, d'un animal et de leurs combinaisons.

5. La composition d'hydrolysat de protéine selon la revendication 1, dans laquelle la composition d'hydrolysat de protéine est dérivée d'une matière protéique à base de soja, et la composition d'hydrolysat de protéine a un degré d'hydrolyse d'environ 0,05 % à environ 35 %.

6. Un procédé permettant d'augmenter l'activité de libération de cholécystokinine d'une cellule, le procédé comprenant la mise en contact de la cellule avec une fraction soluble d'une composition d'hydrolysat de protéine, la composition comprenant un mélange de fragments polypeptidiques ayant une taille moyenne inférieure à environ 100 000 Daltons, ledit mélange comprenant une fraction polypeptidique de 10 000 à 100 000 Daltons, dans lequel une concentration d'au moins environ 0,5 mg/ml de la composition d'hydrolysat de protéine stimule l'activité de libération de cholécystokinine avec une activité sensiblement similaire à plus de 50 % de cholécystokinine libérés par des cellules STC-1 stimulées par 100 nM de 12-myristate-13-acétate de phorbol pendant 4 heures.

7. Le procédé selon la revendication 6, dans lequel la composition d'hydrolysat de protéine a un degré d'hydrolyse d'environ 0,05 % à environ 35 %.

8. Le procédé selon la revendication 6, dans lequel la composition d'hydrolysat de protéine est dérivée d'une matière protéique à base de soja en combinaison avec une matière protéique choisie dans le groupe constitué de l'orge, du canola, du lupin, du maïs, de l'avoine, du pois, de la pomme de terre, du riz, du blé, des oeufs, d'un produit laitier, d'un animal et de leurs combinaisons.

9. Le procédé selon la revendication 6, dans lequel la composition d'hydrolysat de protéine est dérivée d'une matière protéique à base de soja.

10. Le procédé selon la revendication 9, dans lequel la matière protéique à base de soja est choisie dans le groupe constitué d'un extrait de soja, de lait de soja, de poudre de lait de soja, de caillé de soja, de farine de soja dégraissée, de farine de soja partiellement dégraissée, de farine de soja non-dégraissée, de protéine de soja isolée, de concentré de protéine de soja et d'une combinaison de ceux-ci.

11. Le procédé selon la revendication 6, dans lequel la composition d'hydrolysat de protéine de soja est produite en soumettant une matière protéique de départ à une digestion enzymatique.

12. Le procédé selon la revendication 11, dans lequel l'enzyme est une endopeptidase choisie dans le groupe constitué de la sérine protéase (SP1) de Nocardiopsis prasina, de la sérine protéase de Bacillus licheniformis, de la subtilisine protéase de Bacillus licheniformis, de la protéase de type trypsine de Fusarium oxysporum, de la lysyl-endopeptidase d'Achromobacter lyticus, de la subtilisine protéase 2, de la métalloprotéase 1, de l'aspartate protéase 1, de la bromélaïne, de la subtilisine et de leurs combinaisons.

13. Le procédé selon la revendication 12, dans lequel l'enzyme comprend en outre une exopeptidase.

14. Un procédé favorisant la satiété chez un sujet, le procédé comprenant l'administration au sujet d'une quantité d'une fraction soluble d'une composition d'hydrolysat de protéine, la composition comprenant un mélange de fragments polypeptidiques ayant une taille moyenne inférieure à environ 100 000 Daltons, et un degré d'hydrolyse d'environ 0,05 % à environ 35 %, ledit mélange comprenant une fraction polypeptidique de 10 000 à 100 000 Daltons, dans lequel une concentration d'au moins environ 0,5 mg/ml de la composition d'hydrolysat de protéine stimule l'activité de libération de cholécystokinine avec une activité sensiblement similaire à plus de 50 % de cholécystokinine libérés par des cellules STC-1 stimulées par 100 nM de 12-myristate-13-acétate de phorbol pendant 4 heures, dans lequel la quantité administrée procure une sensation de satiété au sujet.

15. Produit alimentaire, le produit alimentaire comprenant :

(a) une matière comestible ; et
(b) une fraction soluble d'une composition d'hydrolysat de protéine, la composition comprenant un mélange de fragments polypeptidiques ayant une taille moyenne inférieure à environ 100 000 Daltons, ledit mélange comprenant une fraction polypeptidique de 10 000 à 100 000 Daltons, dans lequel une concentration d'au moins environ 0,5 mg/ml de la composition d'hydrolysat de protéine stimule l'activité de libération de cholécystokinine avec une activité sensiblement similaire à plus de 50 % de cholécystokinine libérés par des cellules STC-1 stimulées par 100 nM de 12-myristate-13-acétate de phorbol pendant 4 heures.

## Figure 1

CCK Release by STC-1 Cells

Figure 2

Figure 3

EP 2 384 125 B1

Figure 4A

EP 2 384 125 B1

# Figure 4B

**Bromelain**

EP 2 384 125 B1

Figure 4C

# Figure 4D

Alcalase 2

Figure 4E

S2

EP 2 384 125 B1

## Figure 4F

## Figure 4G

Figure 4H

EP 2 384 125 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 61141931 B
- WO 2005035747 A
- US 4266031 A
- US 5874278 A
- US 5459064 A
- WO 0116285 A
- WO 9213964 A
- WO 9113553 A
- WO 9113554 A
- US 5288627 A
- US 5693520 A
- WO 9628542 A

### Non-patent literature cited in the description

- **CORDIER-BUSSAT et al.** M. Peptones Stimulate Cholecystokinin Secretion and Gene Transcription in the Intestinal Cell Line STC-1. *Endocrinology,* 1997, vol. 138 (3), 1137-1144
- **LIDDLE, R.A. et al.** Proteins But Not Amino Acids, Carbohydrates, or Fats Stimulate Cholecystokinin Secretion in the Rat. *Am. J. Physiol.,* 1986, vol. 251, G243-G248
- **SCHASTEEN, C.S. et al.** Correlation of an Immobilized Digestive Enzyme Assay With Poultry True Amino Acid Digestibility for Soybean Meal. *Poultry Science,* 2007, vol. 86 (2), 343-348
- **HIGAKI, N. et al.** *Biosci. Biotechnol. Biochem.,* 2006, vol. 70 (12), 2844-2852
- **NIELSEN, P.M et al.** Improved Method for Determining Food Protein Degree of Hydrolysis. *J. Food Sci.,* 2001, vol. 66 (5), 642-646
- **JENS ADLER-NISSEN.** Determination of the Degree of Hydrolysis of Food Protein Hydrolysates by Trinitrobenzenesulfonic Acid. *J. Agric. Food Chem.,* 1979, vol. 27 (6), 1256-1262
- **RINDI et al.** *Am. J. Pathol.,* 1990, vol. 136, 1349-1364
- **CHANG et al.** *Biochim. Biophys. Acta,* 1994, vol. 1221, 339-437
- **VELLOMEN K-S ; HONKAKOSKI P ; URTTI A.** Substrates and Inhibitors of Efflux Proteins Interfere with the MTT Assay in Cells and May Lead to Underestimation of Drug Toxicity. *Eur. J. Pharm. Sci.,* 2004, vol. 23, 181-188
- **SHASTEEN, C.S. et al.** Correlation of an Immobilized Digestive Enzyme Assay With Poultry True Amino Acid Digestibility for Soybean Meal. *Poultry Science,* 2007, vol. 86 (2), 343-348